# EUROPEAN PATENT SPECIFICATION

(11) **EP 2 637 646 B1**
(45) Date of publication and mention of the grant of the patent: **11.05.2016**
(21) Application number: 11840481.3
(22) Date of filing: 08.11.2011
(51) Int. Cl.: A61K 9/52, A61K 31/554, A61K 31/785, A61P 1/12, A61P 1/16, A61P 3/00

(54) **A PHARMACEUTICAL COMBINATION COMPRISING AN IBAT INHIBITOR AND A BILE ACID BINDER**
PHARMAZEUTISCHE KOMBINATION MIT EINEM IBAT-HEMMER UND EINEM GALLENSÄUREBINDEMITTEL
COMBINAISON PHARMACEUTIQUE COMPRENANT UN INHIBITEUR IBAT ET UN LIANT D'ACIDE BILIAIRE

(30) Priority: 18.11.2010 US 414915 P; 08.11.2010 US 410955 P; 08.11.2010 SE 1051164
(43) Date of publication of application: 18.09.2013
(73) Proprietor: Albireo AB, 413 46 Göteborg (SE)
(72) Inventor: GILLBERG, Per-Göran, S-431 69 Mölndal (SE); GRAFFNER, Hans, S-252 84 Helsingborg (SE); STARKE, Ingemar, S-413 01 Göteborg (SE)
(74) Representative: Novitas Patent AB
(86) International application number: PCT/SE2011/051336
(87) International publication number: WO 2012/064267

(56) References cited:
- WO-A1-00/38728
- WO-A1-00/62810
- WO-A1-02/32428
- WO-A1-03/022286
- WO-A1-03/061663
- WO-A1-03/106482
- WO-A1-2012/064268
- WO-A2-2005/072397
- US-A1- 2005 009 805
- US-A1- 2010 130 472
- Anonymous: "Bowel Diversion Surgeries: Ileostomy, Colostomy, Ileoanal Reservoir, and Continent Ileostomy", NIDDK , February 2009 (2009-02), XP002719261, Retrieved from the Internet: URL:http://digestive.niddk.nih.gov/ddiseas es/pubs/ileostomy/Bowel_Diversion_508.pdf [retrieved on 2014-01-27]
- 'Metabolic Syndrome' THE MERCK MANUAL FOR HEALTH CARE PROFESSIONALS, [Online] October 2008, XP003032721 Retrieved from the Internet: <URL:http://www.merckmanuals.com/profession al/nutritional_disorders/obesity_and_the_me tabolic_syndrome/metabolic_syndrome.html?qt =metabolic_syndrome&alt=sh.> [retrieved on 2012-02-06]
- EUROPEAN ASSOCIATION FOR THE STUDY OF THE LIVER: "EASL Clinical Practice Guidelines: Management of cholestatic liver diseases", JOURNAL OF HEPATOLOGY, ELSEVIER, AMSTERDAM, NL, vol. 51, no. 2, 1 August 2009 (2009-08-01) , pages 237-267, XP026234433, ISSN: 0168-8278, DOI: 10.1016/J.JHEP.2009.04.009 [retrieved on 2009-06-06]

## Description

### FIELD OF THE INVENTION

The present invention relates to a combination comprising a substance with inhibiting effect on the ileal bile acid transport system (IBAT) and at least one other active substance such as a bile acid binder.

### BACKGROUND OF THE INVENTION

It is well known that hyperlipidemic conditions associated with elevated concentrations of total cholesterol and low-density lipoprotein cholesterol are major risk factors for coronary heart disease and particularly artherosclerosis. Interfering with the circulation of bile acids within the lumen of the intestinal tracts is found to reduce the level of cholesterol. Previous established therapies to reduce the concentration of cholesterol involve for instance treatment with HMG-CoA reductase inhibitors, preferably statins such as simvastin and fluvastin, or treatment with bile acid binders, such as resins. Frequently used bile acid binders are for instance cholestyramine, cholestipol and colesevelam. One recently proposed therapy involves the treatment with substances with inhibiting effect on the ileal bile acid transport system (IBAT).

Re-absorption of bile acid from the gastro-intestinal tract is a normal physiological process, which mainly takes place in the ileum by an active transport mechanism called ileal bile acid transport (IBAT). Inhibitors of IBAT can be used in the treatment of hypercholesterolemia. See for instance "Interaction of bile acids and cholesterol with nonsystemic agents having hypocholesterolemic properties", Biochemica et Biophysica Acta, 1210 (1994) 255 - 287. Thus, suitable compounds having such inhibitory IBAT activity are also useful in the treatment of hyperlipidemic conditions.

Several chemical compounds possessing such IBAT activity have recently been described, see for instance hypolipidemic benzothiazepine compounds described in WO 93/16055 and WO 96/16051; condensed 1,4-thiazepines described in WO 94/18183; different heterocyclic compounds described in WO 94/18184; and 1,4-benzothiazepine1,1-dioxides described in WO 96/05188. Further; WO 96/08484; bile acid resorption inhibitors described in WO 97/33882, WO 98/07449, WO 98/03818, WO 98/40375, WO 99/35135, WO 9964409, WO 99/64410, WO 00/01687, WO 00/47568, WO00/61568, DE 19825804, WO 00/38725, WO0038726, WO 00/38727, WO00/38728, WO00/38729, WO01/68096, WO 01/66533, WO 02/50051, WO 02/32428, WO 03/020710, WO 03/022825, WO 03/022830, WO 03/022286, WO 03/061663, WO 03/091232, WO 03/09106482, WO 04/006899, WO 04/076430, WO 07/009655,WO 07009656, WO 08/058630, EP 864582, EP 489423, EP 549 967, EP 573 848, EP 624 593, EP 624 594, EP 624 595, EP 624 596, EP 0864582, EP 1173205.

In general, pharmaceutical drug substances will be absorbed in the upper small intestine, and therefore only a small amount will reach ileum when administered in a conventional oral dosage form. Irrespective of the construction of the pharmaceutical dosage form, it should provide contact for the active compound, e.g. inhibitor of IBAT, with the compound's site of action in the body, for example in the ileum. The above prior art documents discuss in general terms suitable pharmaceutical dosage forms for the described IBAT inhibitor compounds. However, none of the documents describe a specific way to obtain a release of the active substance directly to or close to the site of action. Contact between the active drug and the site of action can be established in different ways.

The inhibition of the re-absorption of bile acids from the small intestine performed by an effective IBAT inhibitor may lead to increased levels of bile acids in the lower parts (colon) of the gastro-intestinal tract. Such an increase of bile acid concentrations in the distal regions could potentially generate diarrhoea and discomfort to the patient. The present invention provides a new approach to minimise the concentration of free bile acids in the colon and thereby reduce the potential risk of adverse events by co-administration of a bile acid binder together with the IBAT inhibitor. However, the combination of an IBAT inhibitor and a bile acid binder has previously been proposed in the above patent applications describing new IBAT inhibitor compounds. The purpose of such previously described combinations has been to enhance the cholesterol lowering efficacy of the therapy. EP1173205 describes that such a combination could be used to minimise a potential risk for diarrhoea connected with IBAT inhibitor therapy.

WO 00/62810 discloses combinations of an IBAT inhibitor and a bile acid binder, for use in the treatment of hypercholesterolaemia. The IBAT inhibitor is formulated for delivery to the ileum and the bile acid binder is formulated for delivery to the colon.

WO 02/32428 discloses a formulation comprising an IBAT inhibitor and an HMG Co-A reductase inhibitor, wherein the IBAT inhibitor is formulated for delivery to the ileum. The formulation may be administered in combination with a bile acid binder, which may be formulated for delivery to the colon. The combination is useful in the treatment of hypercholesterolaemia.

WO 00/38728, WO 03/022286 and WO 03/106482 disclose I BAT inhibitors for use in the treatment of hypercholesterolaemia and hyperlipidemia. The IBAT inhibitors may be administered in combination with a bile acid binder.

WO 03/061663 discloses the use of IBAT inhibitors in the treatment of hypercholesterolaemia and dyslipidaemia, optionally in combination with HMG Co-A reductase inhibitors.

US 2010/130472 discloses the use of IBAT inhibitors in the treatment of obesity and diabetes.

WO 2012/064268 discloses the use of IBAT inhibitors in the treatment of liver diseases. US 2005/0009805 and EP 1535913 disclose the use of 1,4-benzothiazepine derivatives as IBAT inhibitors in the treatment of hyperlipidemia, cholestasis-caused hepatopathy, obesity, fatty liver and steatohepatitis.

### BRIEF DESCRIPTION OF THE INVENTION

The aim of the present invention is to provide a combination for simultaneous, separate or sequential administration which combination comprises an IBAT inhibitor of formula II as disclosed herein and a bile acid binder. Such a combination will protect the patient from any possible side effect caused by excess of bile acids in the colon, such as diarrhoea. If the transport of bile acids is blocked by an IBAT inhibitor the bile acids might be deposited in the colon and induce a secretory diarrhoea as an undesired side effect caused by the treatment with an IBAT inhibitor.

In the provided combination therapy the bile acid binder, for instance a resin such as cholestyramine, cholestipol or colesevelam may be administered in a dosage form with colon release of the bile acid binder. A colon release formulation will provide protection of the bile acid binder to the luminal contents in the more proximal parts of the intestine, where the bile acid concentrations are high. Such a formulation will prevent binding of bile acids to the bile acid binder before the formulation reaches the colon. Thereby, maximal bile acid binding capacity will be obtained in the colon and any possible gastro-intestinal side effects, such as diarrhoea, may be avoided. Thus, any additional amount of bile acid presented in the colon due to the treatment with the IBAT inhibitor compound, would be bound to a bile acid binder, which the bile acid binder is preferably delivered in the colon, thereby any possible side effects such as diarrhoea is avoided and maximal excretion of bile acids will be obtained (IBAT blockade of IBAT will let more bile acids pass to colon where they will be bound to bile acid binders resulting in no passive absorption of bile acids from colon). The release of bile acid binders in colon will decrease the needed dose to archive pharmacological effects from the binder.

A further aspect of the invention is that an increased effect can be achieved by binding the unconjugated bile acids in colon and inhibit the uptake in colon. This leads to a further decrease in bile acid levels and an increase in use of cholesterol for bile acid synthesis, resulting in lower levels of cholesterol in plasma.

Further, the colon stimulating effect of bile acids is limited, which leads to decreases the occurrence of diarrhoea. Moreover, bile acid salts are eluated to a greater extent without affecting the absorption of lipid soluble vitamins A, D, E and K in the small bowel. According to one embodiment an increased efficacy is obtained by using an IBAT inhibitor according to formula (I) or formula (II) including compounds of examples 1-14.

### DETAILED DESCRIPTION OF THE INVENTION

Disclosed herein is a combination designed to deliver the bile acid binder in the colon and the IBAT inhibitor in the small intestine, said combination being intended for the administration of the IBAT inhibitor and the bile acid binder simultaneously, separately or sequentially.

In one aspect, the invention relates to a combination comprising an IBAT inhibitor of formula II as disclosed herein and a bile acid binder for simultaneous, sequential or separate administration, wherein the bile acid binder is formulated for colon release, for use in the treatment of a liver disease selected from the group consisting of primary biliary cirrhosis (PBC); progressive familial intrahepatic cholestasis (PFIC); Alagilles syndrome (ALGS); primary sclerosing cholangitis (PSC); non-alcoholic steatohepatitis (NASH); and pruritus of cholestatic liver disease.

### IBAT inhibitor compounds

IBAT inhibitor compounds are those exhibiting activity when screening for IBAT inhibiting properties. In the literature IBAT inhibitors are often referred to by different names. It is to be understood that where IBAT inhibitors are referred to herein, this term also encompasses compounds known in the literature as: i) ileal apical sodium co-dependent bile acid transporter (ASBT) inhibitors; ii) bile acid transporter (BAT) inhibitors; iii) ileal sodium/bile acid cotransporter system inhibitors; iv) apical sodium-bile acid cotransporter inhibitors; v) ileal sodium-dependent bile acid transport inhibitors; vi) bile acid reabsorption (BARI's) inhibitors; and vii) sodium bile acid transporter (SBAT) inhibitors; where they act by inhibition of IBAT.

Examples of such compounds can be found in the references cited above under the heading "background of the invention and prior art.

Further IBAT inhibitor compounds include benzothiazepines, and more particularly benzothiepines, 1,4-benzothiazepines, 1,5-benzothiazepines, 1,2,5-benzothiadiazepines exhibiting activity when screening for IBAT inhibiting properties.

Other IBAT inhibitors are those in WO 02/50051, WO 03/02286 and WO 03/106482.

Other bile IBAT inhibitors are described in WO9932478, WO0168637, WO03022804, WO0001687 and US 2010/0130472 A1 among those 1-[4-[4-[(4R,5R)-3,3-dibutyl-7-(dimethylamino)-2,3,4,5-tetrahydro-4-hydroxy-1,1-dioxido-1-benzothiepin-5-yl]phenoxy]butyl]4-aza-1-azoniabicyclo[2.2.2]octane methane sulfonate.

Disclosed herein is a combination comprising
(i) A compound of Formula (I) 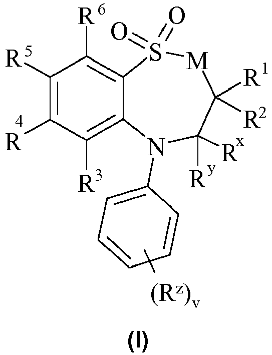 wherein:
   **M** is CH₂, NH
   One of **R¹** and **R²** are selected from hydrogen or C₁₋₆alkyl and the other is selected from C₁₋₆alkyl;
   **R^{x}** and **R^{y}** are independently selected from hydrogen, hydroxy, amino, mercapto, C₁₋₆alkyl, C₁₋₆alkoxy, *N*-(C₁₋₆alkyl)amino, *N*,*N*-(C₁₋₆alkyl)₂amino, C₁₋₆alkylS(O)ₐ wherein a is 0 to 2
   **R^{z}** is selected from halo, nitro, cyano, hydroxy, amino, carboxy, carbamoyl, mercapto, sulphamoyl, C₁₋₆alkyl, C₂₋₆alkenyl, C₂₋₆alkynyl, , C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl and *N*,*N*-(C₁₋₆alkyl)₂Sulphamoyl;
   **v** is 0-5;
   one of **R⁴** and **R⁵** is a group of formula **(IA):** 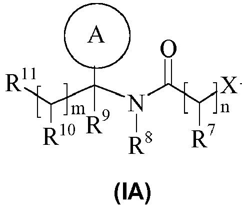
      **R³** and **R⁶** and the other of **R⁴** and **R⁵** are independently selected from hydrogen, halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N*,*N*-(C₁₋₄alkyl)₂amino, C₁₋₄alkanoylamino, *N*-(C₁₋₄alkyl)carbamoyl, *N*,*N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl and *N*,*N*-(C₁₋₄alkyl)₂Sulphamoyl; wherein R³ and R⁶ and the other of R⁴ and R⁵ may be optionally substituted on carbon by one or more R¹⁶;
      **X** is -O-, -N(R^{a})-, -S(O)_{b}- or -CH(R^{a})-; wherein R^{a} is hydrogen or C₁₋₆alkyl and b is 0-2;
      **Ring A** is aryl or heteroaryl; wherein Ring A is optionally substituted by one or more substituents selected from R¹⁷;
      **R⁷** is hydrogen, C₁₋₄alkyl, carbocyclyl or heterocyclyl; wherein R⁷ is optionally substituted by one or more substituents selected from R¹⁸;
      **R⁸** is hydrogen or C₁₋₄alkyl;
      **R⁹** is hydrogen or C₁₋₄alkyl;
      **R¹⁰** is hydrogen, C₁₋₄alkyl, carbocyclyl or heterocyclyl; wherein R¹⁰ is optionally substituted by one or more substituents selected from R¹⁹;
      **R¹¹** is carboxy, sulpho, sulphino, phosphono, -P(O)(OR^{c})(OR^{d}), -P(O)(OH)(OR^{c}), -P(O)(OH)(R^{d}) or -P(O)(OR^{c})(R^{d}) wherein R^{c} and R^{d} are independently selected from C₁₋₆alkyl; or R¹¹ is a group of formula **(IB)** or **(IC):** 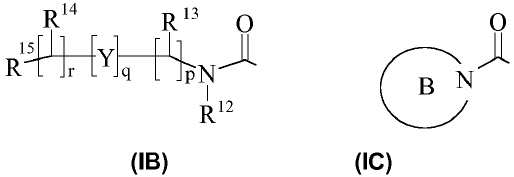 wherein:
         **Y** is -N(Rⁿ)-, -N(Rⁿ)C(O)-, -N(Rⁿ)C(O)(CR^{s}R^{t})ᵥN(Rⁿ)C(O)-, -O-, and -S(O)a-; wherein a is 0-2, v is 1-2, R^{s} and R^{t} are independently selected from hydrogen or C₁₋₄alkyl optionally substituted by R²⁶ and Rⁿ is hydrogen or C₁₋₄alkyl;
         **R¹²** is hydrogen or C₁₋₄alkyl;
         **R¹³** and **R¹⁴** are independently selected from hydrogen, C₁₋₄alkyl, carbocyclyl or heterocyclyl; and when q is 0, R¹⁴ may additionally be selected from hydroxy wherein R¹³ and R¹⁴ may be independently optionally substituted by one or more substituents selected from R²⁰;
         **R¹⁵** is carboxy, sulpho, sulphino, phosphono, -P(O)(OR^{e})(OR^{f}), -P(O)(OH)(OR^{e}), -P(O)(OH)(R^{e}) or -P(O)(OR^{e})(R^{f}) wherein R^{e} and R^{f} are independently selected from C₁₋₆alkyl;
         **p** is 1-3; wherein the values of R¹³ may be the same or different;
         **q** is 0-1;
         **r** is 0-3; wherein the values of R¹⁴ may be the same or different;
         **m** is 0-2; wherein the values of R¹⁰ may be the same or different;
         **n** is 1-3; wherein the values of R⁷ may be the same or different;
         **Ring B** is a nitrogen linked heterocyclyl substituted on carbon by one group selected from R²³, and optionally additionally substituted on carbon by one or more R²⁴; and wherein if said nitrogen linked heterocyclyl contains an -NH- moiety, that nitrogen may be optionally substituted by a group selected from R²⁵;
         **R¹⁶, R¹⁷** and **R¹⁸** are independently selected from halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N*,*N*-(C₁₋₄alkyl)₂amino, C₁₋₄alkanoylamino, *N*-(C₁₋₄alkyl)carbamoyl, *N*,*N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl and *N*,*N*-(C₁₋₄alkyl)₂Sulphamoyl; wherein R¹⁶, R¹⁷ and R¹⁸ may be independently optionally substituted on carbon by one or more R²¹;
         **R¹⁹, R²⁰, R²⁴** and **R²⁶** are independently selected from halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N*,*N*-(C₁₋₄alkyl)₂amino, C₁₋₄alkanoylamino, *N*-(C₁₋₄alkyl)carbamoyl, *N*,*N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl, *N*,*N*-(C₁₋₄alkyl)₂sulphamoyl, carbocyclyl, heterocyclyl, benzyloxycarbonylamino, sulpho, sulphino, amidino, phosphono, -P(O)(OR^{a})(OR^{b}), -P(O)(OH)(OR^{a}), -P(O)(OH)(R^{a}) or -P(O)(OR^{a})(R^{b}), wherein R^{a} and R^{b} are independently selected from C₁₋₆alkyl; wherein R¹⁹, R²⁰ R²⁴ and R²⁶ may be independently optionally substituted on carbon by one or more R²²;
         **R²¹** and **R²²** are independently selected from halo, hydroxy, cyano, carbamoyl, ureido, amino, nitro, carboxy, carbamoyl, mercapto, sulphamoyl, trifluoromethyl, trifluoromethoxy, methyl, ethyl, methoxy, ethoxy, vinyl, allyl, ethynyl, methoxycarbonyl, formyl, acetyl, formamido, acetylamino, acetoxy, methylamino, dimethylamino, *N*-methylcarbamoyl, *N*,*N*-dimethylcarbamoyl, methylthio, methylsulphinyl, mesyl, *N*-methylsulphamoyl and *N*,*N*-dimethylsulphamoyl;
         **R²³** is carboxy, sulpho, sulphino, phosphono, -P(O)(OR^{g})(OR^{h}), -P(O)(OH)(OR^{g}), -P(O)(OH)(R^{g}) or -P(O)(OR^{g})(R^{h}) wherein R^{g} and R^{h} are independently selected from C₁₋₆alkyl;
         **R²⁵** is selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N*,*N*-(C₁₋₆alkyl)carbamoyl, benzyl, benzyloxycarbonyl, benzoyl and phenylsulphonyl;
   or a pharmaceutically acceptable salt thereof; and
(ii) at least one other active substance selected from an IBAT inhibitor; an enteroendocrine peptide or enhancer thereof; a dipeptidyl peptidase-IV inhibitor; a biguanidine; an incretin mimetic; a thiazolidinone; a PPAR agonist; a HMG Co-A reductase inhibitor; a bile acid binder; and a TGR5 receptor modulator; or a pharmaceutically acceptable salt of any one of said active substances;
   wherein the compound of formula (I) and the at least one other active substance is administered simultaneously, sequentially or separately.

In this specification the term "alkyl" includes both straight and branched chain alkyl groups but references to individual alkyl groups such as "propyl" are specific for the straight chain version only. For example, "C₁₋₆alkyl" includes C₁₋₄alkyl, C₁₋₃alkyl, propyl, isopropyl and *t*-butyl. However, references to individual alkyl groups such as 'propyl' are specific for the straight chained version only and references to individual branched chain alkyl groups such as 'isopropyl' are specific for the branched chain version only. A similar convention applies to other radicals, for example "phenylC₁₋₆alkyl" would include phenylC₁₋₄alkyl, benzyl, 1-phenylethyl and 2-phenylethyl. The term "halo" refers to fluoro, chloro, bromo and iodo.

Where optional substituents are selected from "one or more" groups it is to be understood that this definition includes all substituents being selected from one of the specified groups or the substituents being selected from two or more of the specified groups.

"Heteroaryl" is a totally unsaturated, mono or bicyclic ring containing 3-12 atoms of which at least one atom is selected from nitrogen, sulphur or oxygen, which may, unless otherwise specified, be carbon or nitrogen linked. Preferably "heteroaryl" refers to a totally unsaturated, monocyclic ring containing 5 or 6 atoms or a bicyclic ring containing 9 or 10 atoms of which at least one atom is selected from nitrogen, sulphur or oxygen, which may, unless otherwise specified, be carbon or nitrogen linked. In another aspect, "heteroaryl" refers to a totally unsaturated, monocyclic ring containing 5 or 6 atoms or a bicyclic ring containing 8, 9 or 10 atoms of which at least one atom is selected from nitrogen, sulphur or oxygen, which may, unless otherwise specified, be carbon or nitrogen linked. Examples and suitable values of the term "heteroaryl" are thienyl, isoxazolyl, imidazolyl, pyrrolyl, thiadiazolyl, isothiazolyl, triazolyl, pyranyl, indolyl, pyrimidyl, pyrazinyl, pyridazinyl, pyridyl and quinolyl. Preferably the term "heteroaryl" refers to thienyl or indolyl.

"Aryl" is a totally unsaturated, mono or bicyclic carbon ring that contains 3 - 12 atoms. Preferably "aryl" is a monocyclic ring containing 5 or 6 atoms or a bicyclic ring containing 9 or 10 atoms. Suitable values for "aryl" include phenyl or naphthyl. Particularly "aryl" is phenyl.

A "heterocyclyl" is a saturated, partially saturated or unsaturated, mono or bicyclic ring containing 3 - 12 atoms of which at least one atom is selected from nitrogen, sulphur or oxygen, which may, unless otherwise specified, be carbon or nitrogen linked, wherein a -CH₂- group can optionally be replaced by a -C(O)- or a ring sulphur atom may be optionally oxidised to form the S-oxides. Preferably a "heterocyclyl" is a saturated, partially saturated or unsaturated, mono or bicyclic ring containing 5 or 6 atoms of which at least one atom is selected from nitrogen, sulphur or oxygen, which may, unless otherwise specified, be carbon or nitrogen linked, wherein a -CH₂-group can optionally be replaced by a -C(O)- or a ring sulphur atom may be optionally oxidised to form S-oxide(s). Examples and suitable values of the term "heterocyclyl" are thiazolidinyl, pyrrolidinyl, pyrrolinyl, 2-pyrrolidonyl, 2,5-dioxopyrrolidinyl, 2-benzoxazolinonyl, 1,1-dioxotetra-hydrothienyl, 2,4-dioxoimidazolidinyl, 2-oxo-1,3,4-(4-triazolinyl), 2-oxazolidinonyl, 5,6-dihydrouracilyl, 1,3-benzodioxolyl, 1,2,4-oxadiazolyl, 2-azabicyclo [2.2.1] heptyl, 4-thiazolidonyl, morpholino, 2-oxotetrahydrofuranyl, tetrahydrofuranyl, 2,3-dihydro-benzofuranyl, benzothienyl, tetrahydropyranyl, piperidyl, 1-oxo-1,3-dihydroisoindolyl, piperazinyl, thiomorpholino, 1,1-dioxothiomorpholino, tetrahydropyranyl, 1,3-dioxolanyl, homopiperazinyl, thienyl, isoxazolyl, imidazolyl, pyrrolyl, thiadiazolyl, isothiazolyl, 1,2,4-triazolyl, 1,3,4-triazolyl, pyranyl, indolyl, pyrimidyl, thiazolyl, pyrazinyl, pyridazinyl, pyridyl, 4-pyridonyl, quinolyl and 1-isoquinolonyl.

A "carbocyclyl" is a saturated, partially saturated or unsaturated, mono or bicyclic carbon ring that contains 3 - 12 atoms; wherein a -CH₂- group can optionally be replaced by a - C(O)-. Preferably "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl. Particularly "carbocyclyl" is cyclopropyl, cyclobutyl, 1-oxocyclopentyl, cyclopentyl, cyclopentenyl, cyclohexyl, cyclohexenyl, phenyl or 1-oxoindanyl.

An example of "C₁₋₆alkanoyloxy" and "C₁₋₄alkanoyloxy" is acetoxy. Examples of "C₁₋₆alkoxycarbonyl" and "C₁₋₄alkoxycarbonyl" include methoxycarbonyl, ethoxycarbonyl, *n-* and *t*-butoxycarbonyl. Examples of "C₁₋₆alkoxy" and "C₁₋₄alkoxy" include methoxy, ethoxy and propoxy. Examples of "C₁₋₆alkanoylamino" and "C₁₋₄alkanoylamino" include formamido, acetamido and propionylamino. Examples of "C₁₋₆alkylS(O)ₐ wherein a is 0 to 2" and "C₁₋₄alkylS(O)ₐ wherein a is 0 to 2" include methylthio, ethylthio, methylsulphinyl, ethylsulphinyl, mesyl and ethylsulphonyl. Examples of "C₁₋₆alkanoyl" and "C₁₋₄alkanoyl" include C₁₋₃alkanoyl, propionyl and acetyl. Examples of "*N*-(C₁₋₆alkyl)amino" and "*N*-(C₁₋₄alkyl)amino" include methylamino and ethylamino. Examples of "*N*,*N*-(C₁₋₆alkyl)2amino" and "*N*,*N*-(C₁₋₄alkyl)₂amino" include di-*N*-methylamino, di-(*N*-ethyl)amino and *N*-ethyl-*N*-methylamino. Examples of "C₂₋₆alkenyl" and "C₂₋₄alkenyl" are vinyl, allyl and 1-propenyl. Examples of "C₂₋₆alkynyl" and "C₂₋₄alkynyl" are ethynyl, 1-propynyl and 2-propynyl. Examples of "*N*-(C₁₋₆alkyl)sulphamoyl" and "*N*-(C₁₋₄alkyl)-sulphamoyl" are *N*-(C₁₋₃alkyl)sulphamoyl, *N*-(methyl)sulphamoyl and *N*-(ethyl)-sulphamoyl. Examples of "*N*-(C₁₋₆alkyl)₂sulphamoyl" and "*N*-4alkyl)₂sulphamoyl" are *N,N-*(dimethyl)sulphamoyl and *N*-(methyl)-*N*-(ethyl)sulphamoyl. Examples of "*N*-(C₁₋₆alkyl)-carbamoyl" and "*N*-(C₁₋₄alkyl)carbamoyl" are methylaminocarbonyl and ethylaminocarbonyl. Examples of "*N*,*N*-(C₁₋₆alkyl)₂carbamoyl" and "*N*,*N*-(C₁₋₄alkyl)₂ carbamoyl" are dimethylaminocarbonyl and methylethylaminocarbonyl. Examples of "C₁₋₆alkoxy-carbonylamino" are ethoxycarbonylamino and *t*-butoxycarbonylamino. Examples of"*N*"-(C₁₋₆alkyl)ureido" are *N*-methylureido and *N*-ethylureido. Examples of "*N*-(C₁₋₆alkyl)ureido are *N*-methylureido and *N*-ethylureido. Examples of "*N*',*N*'-(C₁₋₆alkyl)₂-ureido are *N',N'-*dimethylureido and *N*'-methyl-*N*'-ethylureido. Examples of "*N*-(C₁₋₆alkyl)-*N*-(C₁₋₆alkyl)-ureido are *N*'-methyl-*N*-methylureido and *N*-propyl-*N*-methylureido. Examples of *"N*'*,N*'-(C₁₋₆alkyl)₂-*N*-(C₁₋₆alkyl)ureido are *N'*,*N'*-dimethyl-*N*-methylureido and *N*-methyl-*N*-ethyl-*N*-propylureido.

A suitable pharmaceutically acceptable salt of a compound is, for example, an acid-addition salt of a compound which is sufficiently basic, for example, an acid-addition salt with, for example, an inorganic or organic acid, for example hydrochloric, hydrobromic, sulphuric, phosphoric, trifluoroacetic, citric or maleic acid.

In addition a suitable pharmaceutically acceptable salt of a compound which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl) amine.

It is also to be understood that certain compounds of the formula **(I)** can exist in solvated as well as unsolvated forms such as, for example, hydrated forms. It is to be understood that formula **(I)** encompasses all such solvated forms which possess IBAT inhibitory activity.

Preferred values of **R¹, R², R³, R⁴, R⁵** and **R⁶** are as follows. Such values may be used where appropriate with any of the definitions, claims or embodiments defined hereinbefore or hereinafter.

Preferably **R¹** and **R²** are independently selected from C₁₋₄alkyl.
More preferably **R¹** and **R²** are independently selected from ethyl or butyl.
More preferably **R¹** and **R²** are independently selected from ethyl, propyl or butyl.
In one aspect particularly **R¹** and **R²** are both butyl.
In a further aspect particularly **R¹** and **R²** are both propyl.
In another aspect particularly one of **R¹** and **R²** is ethyl and the other is butyl.

Preferably **R^{x}** and **R^{Y}** are independently selected from hydrogen or C₁₋₆alkyl.
More preferably **R^{x}** and **R^{Y}** are both hydrogen.
Preferably **R^{z}** is selected from halo, amino, C₁₋₆alkyl, C₁₋₆alkoxycarbonylamino or *N*'-(C₁₋₆alkyl)ureido.

More preferably **R^{z}** is selected from chloro, amino, *t*-butyl, *t*-butoxycarbonylamino or *N'*-(*t*-butyl)ureido.

Preferably **v** is 0 or 1.
In one aspect, more preferably **v** is 0.
In one aspect, more preferably **v** is 1.
In one aspect preferably **R⁴** is a group of formula **(IA)** (as depicted above).
In another aspect preferably **R⁵** is a group of formula **(IA)** (as depicted above).

Preferably **R³** and **R⁶** are hydrogen.

Preferably the other of **R⁴** and **R⁵** that is not the group of formula **(IA)** is selected from halo, C₁₋₄alkoxy or C₁₋₄alkylS(O)ₐ wherein a is 0 to 2; wherein that **R⁴** or **R⁵** may be optionally substituted on carbon by one or more **R¹⁶;** wherein **R¹⁶** is independently selected from hydroxy and *N*,*N*-(C₁₋₄alkyl)₂amino.

More preferably the other of **R⁴** and **R⁵** that is not the group of formula **(IA)** is selected from bromo, methoxy, isopropoxy, methylthio, ethylthio, isopropylthio or mesyl; wherein that **R⁴** or **R⁵** may be optionally substituted on carbon by one or more **R¹⁶;** wherein **R¹⁶** is independently selected from hydroxy and *N*,*N*-dimethylamino.

Particularly the other of **R⁴** and **R⁵** that is not the group of formula **(IA)** is selected from bromo, methoxy, isopropoxy, methylthio, ethylthio, isopropylthio, 2-hydroxyethylthio, 2-(N,N-dimethylamino) ethylthio or mesyl.

More particularly the other of **R⁴** and **R⁵** that is not the group of formula **(IA)** is methylthio. Preferably the other of **R⁴** and **R⁵** that is not the group of formula **(IA)** is selected from hydrogen, halo, C₁₋₄alkoxy or C₁₋₄alkylS(O)ₐ wherein a is 0 to 2; wherein that **R⁴** or **R⁵** may be optionally substituted on carbon by one or more **R¹⁶;** wherein **R¹⁶** is independently selected from hydroxy, carboxy and *N*,*N*-(C₁₋₄alkyl)₂amino.

More preferably the other of **R⁴** and **R⁵** that is not the group of formula **(IA)** is selected from hydrogen, bromo, methoxy, isopropoxy, methylthio, ethylthio, isopropylthio or mesyl; wherein that **R⁴** or **R⁵** may be optionally substituted on carbon by one or more **R¹⁶;** wherein **R¹⁶** is independently selected from hydroxy, carboxy and *N*,*N*-dimethylamino.

Particularly the other of **R⁴** and **R⁵** that is not the group of formula **(IA)** is selected from hydrogen, bromo, methoxy, isopropoxy, methylthio, carboxymethylthio, ethylthio, isopropylthio, 2-hydroxyethylthio, 2-(*N*,*N*-dimethylamino) ethylthio or mesyl.
In another aspect, more preferably the other of **R⁴** and **R⁵** that is not the group of formula **(IA)** is selected from hydrogen, chloro, bromo, methoxy, isopropoxy, methylthio, ethylthio or isopropylthio; wherein that **R⁴** or **R⁵** may be optionally substituted on carbon by one or more **R¹⁶;** wherein **R¹⁶** is independently selected from hydroxy, carboxy and *N,N-*dimethylamino.

In another aspect, particularly the other of **R⁴** and **R⁵** that is not the group of formula **(IA)** is selected from hydrogen, chloro, bromo, methoxy, isopropoxy, methylthio, carboxymethylthio, ethylthio, isopropylthio, 2-hydroxyethylthio or 2-(*N*,*N*-dimethylamino) ethylthio.

In another aspect, more particularly the other of **R⁴** and **R⁵** that is not the group of formula **(IA)** is bromo or chloro.
In another aspect, more particularly the other of **R⁴** and **R⁵** that is not the group of formula **(IA)** is methoxy.

In one aspect, preferably **Ring A** is aryl.
In another aspect, preferably **Ring A** is heteroaryl.
When **Ring A** is aryl, preferably **Ring A** is phenyl.
When **Ring A** is heteroaryl, preferably **Ring A** is thienyl or indolyl.
Preferably **Ring A** is aryl or heteroaryl; wherein **Ring A** is optionally substituted by one or more substituents selected from **R¹⁷;** wherein **R¹⁷** is selected from halo, hydroxy or C₁₋₄alkyl; wherein **R¹⁷** may be optionally substituted on carbon by one or more **R²¹;** wherein **R²¹** is selected from halo.
Preferably X is-O.

More preferably **Ring A** is phenyl, thienyl or indolyl; wherein **Ring A** is optionally substituted by one or more substituents selected from halo, hydroxy or trifluoromethyl. Particularly **Ring A** is selected from phenyl, 4-hydroxyphenyl, thien-2-yl, 4-trifluoromethylphenyl, 3-hydroxyphenyl, 2-fluorophenyl, 2,3-dihydroxyphenyl or indol-3-yl.
More particularly **Ring A** is phenyl.

In another aspect, preferably **Ring A** is aryl or heteroaryl; wherein **Ring A** is optionally substituted by one or more substituents selected from **R¹⁷;** wherein **R¹⁷** is selected from halo, hydroxy, C₁₋₄alkyl or C₁₋₄alkoxy; wherein **R¹⁷** may be optionally substituted on carbon by one or more **R²¹;** wherein **R²¹** is selected from halo.

In another aspect, more preferably **Ring A** is phenyl, thienyl or indolyl; wherein **Ring A** is optionally substituted by one or more substituents selected from halo, hydroxy, methoxy or trifluoromethyl.

In another aspect, particularly **Ring A** is selected from phenyl,
4-hydroxyphenyl, 4-methoxyphenyl, thien-2-yl, 4-trifluoromethylphenyl, 3-hydroxyphenyl, 2-fluorophenyl, 2,3-dihydroxyphenyl or indol-3-yl.
In a further aspect, particularly **Ring A** is selected from phenyl,
4-hydroxyphenyl, 4-methoxyphenyl, thien-2-yl, 4-trifluoromethylphenyl, 3-hydroxyphenyl, 2-fluorophenyl, 4-fluorophenyl, 2,3-dihydroxyphenyl or indol-3-yl.

Preferably **R⁷** is hydrogen, C₁₋₄alkyl or carbocyclyl.
More preferably **R⁷** is hydrogen, methyl or phenyl.
Particularly **R⁷** is hydrogen.
In one aspect, preferably **R⁸** is hydrogen.
In another aspect, preferably **R⁸** is C₁₋₄alkyl.
In another aspect, more preferably **R⁸** is hydrogen or methyl.
In one aspect, preferably **R⁹** is hydrogen.
In another aspect, preferably **R⁹** is C₁₋₄alkyl.
In another aspect, more preferably **R⁹** is hydrogen or methyl.
Preferably **R¹⁰** is hydrogen.
In one aspect, preferably **R¹¹** is carboxy, sulpho, sulphino, phosphono, -P(O)(OR^{c})(OR^{d}), P(O)(OH)(OR^{c}), -P(O)(OH)(R^{d}) or -P(O)(OR^{c}) (R^{d}) wherein R^{c} and R^{d} are independently selected from C₁₋₆alkyl.

In another aspect, preferably **R¹¹** is a group of formula **(IB)** (as depicted above).

Preferably **R¹¹** is carboxy, -P(O)(OH)(OR^{c}) or a group of formula **(IB)** (as depicted above). More preferably **R¹¹** is carboxy, -P(O)(OH)(OEt) or a group of formula **(IB)** (as depicted above).

In another aspect, preferably **R¹¹** is carboxy, sulpho, -P(O)(OH)(OR^{c}) wherein R^{c} is selected from C₁₋₄alkyl or a group of formula **(IB)** (as depicted above).
Preferably **Y** is -NH- or -NHC (O)-.
More preferably **Y** is -NHC (O)-.
In one aspect, preferably **R¹²** is hydrogen.
In another aspect, preferably **R¹²** is C₁₋₄alkyl.
In another aspect, more preferably **R¹²** is hydrogen or methyl.
Preferably **R¹³** is hydrogen, C₁₋₄alkyl or carbocyclyl; wherein **R¹³** is optionally substituted by one or more substituents selected from **R²⁰;** wherein **R²⁰** is hydroxy.
More preferably **R¹³** is hydrogen, methyl or phenyl; wherein **R¹³** is optionally substituted by one or more substituents selected from **R²⁰;** wherein **R²⁰** is hydroxy.
Particularly **R¹³** is hydrogen, hydroxymethyl or phenyl.
More particularly **R¹³** is hydrogen or hydroxymethyl.

In another aspect, preferably **R¹³** is hydrogen, C₁₋₄alkyl or carbocyclyl; wherein **R¹³** is optionally substituted by one or more substituents selected from **R²⁰;** wherein **R²⁰** is hydroxy, carboxy, carbocyclyl or amino; wherein **R²⁰** may be optionally substituted on carbon by one or more **R²²; R²²** is hydroxy.

In another aspect, more preferably **R¹³** is hydrogen, methyl, ethyl, butyl or phenyl; wherein **R¹³** is optionally substituted by one or more substituents selected from **R²⁰;** wherein **R²⁰** is hydroxy, carboxy, phenyl or amino; wherein **R²⁰** may be optionally substituted on carbon by one or more **R²²; R²²** is hydroxy.

In another aspect, particularly **R¹³** is hydrogen, hydroxymethyl, 4-aminobutyl, 2-carboxyethyl, 4-hydroxybenzyl or phenyl.

In a further aspect, preferably **R¹³** is hydrogen, C₁₋₄alkyl or carbocyclyl; wherein **R¹³** is optionally substituted by one or more substituents selected from **R²⁰;** wherein **R²⁰** is hydroxy, carboxy, carbocyclyl, heterocyclyl or amino; wherein **R²⁰** may be optionally substituted on carbon by one or more **R²²;** R²² is hydroxy.

In a further aspect, more preferably **R¹³** is hydrogen, methyl, ethyl, butyl or phenyl; wherein **R¹³** is optionally substituted by one or more substituents selected from **R²⁰;** wherein **R²⁰** is hydroxy, carboxy, phenyl, imidazolyl or amino; wherein **R²⁰** may be optionally substituted on carbon by one or more **R²²; R²²** is hydroxy.

In a further aspect, particularly **R¹³** is hydrogen, hydroxymethyl, 4-aminobutyl, 2-carboxyethyl, 4-hydroxybenzyl, imidazol-5-ylmethyl or phenyl.

In another further aspect, preferably **R¹³** is hydrogen, C₁₋₄alkyl, carbocyclyl or **R²³;** wherein **R¹³** is optionally substituted by one or more substituents selected from **R²⁰;** wherein **R²⁰** is hydroxy, C₁₋₄alkylS (O) a wherein a is 0, C₁₋₄alkoxy, amino, carbocyclyl, heterocyclyl or mercapto; wherein **R²⁰** may be independently optionally substituted on carbon by one or more **R²²; R²²** is selected from hydroxy; and **R²³** is carboxy.

In another further aspect, more preferably **R¹³** is hydrogen, methyl, ethyl, butyl or phenyl or **R²³;** wherein **R¹³** is optionally substituted by one or more substituents selected from **R²⁰;** wherein **R²⁰** is hydroxy, methylthio, methoxy, amino, imidazolyl or mercapto; wherein **R²⁰** may be independently optionally substituted on carbon by one or more **R²²; R²²** is selected from hydroxy; and **R²³** is carboxy.

In another further aspect, particularly **R¹³** is hydrogen, carboxy, hydroxymethyl, mercaptomethyl, methoxymethyl, methylthiomethyl, 2-methylthioethyl, 4-aminobutyl, 4-hydroxybenzyl, imidazol-5-ylmethyl or phenyl.

In another aspect more particularly **R¹³** is methylthiomethyl, methylsulphinylmethyl or methylsulphonylmethyl.

Preferably **R¹⁴** is hydrogen.

In another aspect, preferably **R¹⁴** is selected from hydrogen, C₁₋₄alkyl or carbocyclyl; wherein said C₁₋₄alkyl or carbocyclyl may be optionally substituted by one or more substituents selected from **R²⁰;** and **R²⁰** is hydroxy.
In another aspect, more preferably **R¹⁴** is selected from hydrogen, methyl or phenyl; wherein said methyl or phenyl may be optionally substituted by one or more substituents selected from **R²⁰;** and **R²⁰** is hydroxy.

In another aspect, particularly **R¹⁴** is hydrogen, phenyl or hydroxymethyl.
Particularly **R¹⁵** is carboxy or sulpho.

In one aspect, more particularly **R¹⁵** is carboxy.
In another aspect, more particularly **R¹⁵** is sulpho.

Preferably **R¹⁵** is carboxy, sulpho,-P(O)(OR^{e}) (OR^{f}), -P(O)(OH)(OR^{e}), -P(O)(OH)(R^{e}) or -P(O)(OR^{e})(R^{f}) wherein R^{e} and R^{f} are independently selected from C₁₋₄alkyl.
More preferably **R¹⁵** is carboxy, sulpho, -P(O)(OR^{e})(OR^{f}), -P(O)(OH)(OR^{e}), -P(O)(OH)(R^{e}) or -P(O)(OR^{e})(R^{f}) wherein R^{e} and R^{f} are independently selected from methyl or ethyl. Preferably **R¹⁵** is carboxy, sulpho, -P(O)(OEt)(OEt), -P(O)(OH)(OEt), -P(O)(OH)(Me) or -P (O)(OEt)(Me).

Preferably **R¹⁵** is carboxy, sulpho, phosphono, -P(O)(OR^{e})(OR^{f}), -P(O)(OH)(OR^{e}),-P(O)(OH) (R^{e}) or -P(O)(OR^{e})(R^{f}) wherein R^{e} and R^{f} are independently selected from C₁₋₄alkyl or **R¹⁵** is a group of formula **(IC)** (as depicted above).

More preferably **R¹⁵** is carboxy, sulpho, phosphono,-P(O)(OR^{e})(OR^{f}), -P(O)(OH)(OR^{e}), -P(O)(OH)(R^{e}) or -P(O)(OR^{e})(R^{f}) wherein R^{e} and R^{f} are independently selected from methyl or ethyl or **R¹⁵** is a group of formula **(IC)** (as depicted above).

Preferably **R¹⁵** is carboxy, sulpho, phosphono, -P(O)(OEt)(OEt), -P (O)(O*t*-Bu)(O*t*-Bu), -P(O)(OH)(OEt), -P (O)(OH)(Me) or-P(O)(OEt)(Me) or **R¹⁵** is a group of formula **(IC)** (as depicted above).

In one aspect, preferably **R¹⁵** is carboxy.

In another aspect, preferably **R¹⁵** is sulpho.
In another aspect, preferably **R¹⁵** is -P(O)(OH)(OEt).
In another aspect, preferably **R¹⁵** is -P(O)(OH)(Me).
In another aspect, preferably **R¹⁵** is -P(O)(OEt)(Me).
In one aspect, preferably **R²⁴** is hydrogen.
In another aspect, preferably **R²⁴** is C₁₋₄alkyl.

Preferably **R²⁵** is hydrogen.
Preferably **R²⁶** is carboxy.

Preferably **p** is 1 or 2; wherein the values of **R¹³** may be the same or different.
In one aspect, more preferably **p** is 1.
In another aspect, more preferably **p** is 2; wherein the values of **R¹³** may be the same or different.
In a further aspect, more preferably **p** is 3; wherein the values of **R¹³** may be the same or different.
In one aspect, preferably **q** is 0.
In a further aspect, preferably **q** is 1.
In one aspect, preferably **r** is 0.
In one aspect, more preferably **r** is 1.
In another aspect, more preferably **r** is 2; wherein the values of **R¹⁴** may be the same or different.
In a further aspect, more preferably **r** is 3; wherein the values of **R¹⁴** may be the same or different.
Preferably **m** is 0.
In another aspect, preferably **m** is 0 or 1.
Preferably **n** is 1.
In another aspect, preferably **n** is 1 or 2.
Preferably **z** is **1.**
The group of formula **(IA)** wherein **R⁷** is hydrogen, methyl or phenyl, **n** is 1, **Ring A** is phenyl, thienyl or indolyl; wherein **Ring A** is optionally substituted by one or more substituents selected from halo, hydroxy or trifluoromethyl, **m** is 0 and R⁹ is carboxy,-P(O)(OH)(OR^{c}) or a group of formula **(IB).**
The group of formula **(IA)** wherein: X is -0-.
**Ring A** is phenyl, thienyl or indolyl; wherein **Ring A** is optionally substituted by one or more substituents selected from halo, hydroxy, methoxy or trifluoromethyl;
**R⁷** is hydrogen, methyl or phenyl;
**R⁸** is hydrogen or methyl;
**R⁹** is hydrogen or methyl;
**R¹⁰** is hydrogen;
**m** is 0-2 wherein the values of **R¹⁰** may be the same or different; and **R¹¹** is carboxy, -P(O)(OH)(OEt) or a group of formula **(IB)** (as depicted in claim 1); The group of formula **(IB)** wherein **R¹⁰** is hydrogen, hydroxymethyl or phenyl, **p** is 1 or 2; wherein the values of **R¹⁰** may be the same or different and **R¹¹** is carboxy or sulpho.
The group of formula **(IB)** wherein:
**R¹²** is hydrogen or methyl;
**R¹³** is hydrogen, methyl, ethyl, butyl or phenyl or **R²³;** wherein **R¹³** is optionally substituted by one or more substituents selected from **R²⁰; R²⁰** is hydroxy, methylthio, methoxy, amino, imidazolyl or mercapto; wherein **R²⁰** may be independently optionally substituted on carbon by one or more hydroxy; **R²³** is carboxy; **Y** is -NH- or -NHC (O)-; **R¹⁴** is selected from hydrogen, methyl or phenyl; wherein said methyl or phenyl may be optionally substituted by one or more substituents selected from hydroxy; **R¹⁵** is carboxy, sulpho, phosphono, -P(O)(OR^{e})(OR^{f}), -P(O)(OH)(OR^{e}), -P(O)(OH)(R^{e}) or -P(O)(OR^{e})(R^{f}) wherein R^{e} and R^{f} are independently selected from methyl or ethyl or **R¹⁵** is a group of formula **(IC)** (as depicted in claim 1);
**p** is 1-3 wherein the values of **R¹³** may be the same or different;
**q** is 0-1; and
**r** is 0-3 wherein the values of **R¹⁴** may be the same or different;
The group of formula **(IC)** wherein
**R²⁴** is hydrogen;
**R²⁵** is hydrogen;
**R²⁶** is carboxy; and
**z** is 1;
or a pharmaceutically acceptable salt thereof.

Therefore in a further aspect, there is provided a compound of formula **(I)** as depicted above wherein:
**R¹** and **R²** are independently selected from ethyl or butyl;
**R³** and **R⁶** are hydrogen;
**R⁴** is selected from halo, C₁₋₄alkoxy or C₁₋₄alkylS(O)ₐ wherein a is 0 to 2; wherein that **R⁴** may be optionally substituted on carbon by one or more **R¹⁶;** wherein **R¹⁶** is independently selected from hydroxy and *N*,*N*-(C₁₋₄alkyl)₂amino;
**R⁵** is a group of formula **(IA);**
**Ring A** is aryl or heteroaryl; wherein **Ring A** is optionally substituted by one or more substituents selected from **R¹⁷;** wherein
**R¹⁷** is selected from halo, hydroxy or C₁₋₄alkyl; wherein **R¹⁷** may be optionally substituted on carbon by one or more **R²¹;** wherein
**R²¹** is selected from halo;
**R⁷** is hydrogen, C₁₋₄alkyl or carbocyclyl;
**R¹¹** is carboxy, -P(O)(OH)(OR^{c}) or a group of formula **(IB)** (as depicted above);
**R¹³** is hydrogen, C₁₋₄alkyl or carbocyclyl; wherein **R¹³** is optionally substituted by one or more substituents selected from **R²⁰;** wherein
**R²⁰** is hydroxy;
**R¹⁵** is carboxy or sulpho;
**p** is 1 or 2; wherein the values of **R¹³** may be the same or different;
**m** is 0; and
**n** is 1;
or a pharmaceutically acceptable salt thereof.

Therefore in an additional aspect, there is provided a compound of formula (I) as depicted above wherein:
**R¹** and **R²** are both butyl or one of **R¹** and **R²** is ethyl and the other is butyl;
**R⁴** is methylthio;
**R⁵** is a group of formula **(IA)** (as depicted above);
**R³** and **R⁶** are hydrogen;
**Ring A** is phenyl;
**R⁷** is hydrogen;
**R¹¹** is a group of formula **(IB)** (as depicted above);
**R¹³** is hydrogen or hydroxymethyl;
**R¹⁵** is carboxy or sulpho;
**p** is 1 or 2; wherein the values of **R¹³** may be the same or different;
**m** is 0;
**n** is 1;
or a pharmaceutically acceptable salt thereof.

Therefore in an additional further aspect, there is provided a compound of formula **(I)** as depicted above wherein:
**R¹** and **R²** are independently selected from ethyl or butyl;
**R³** and **R⁶** are hydrogen;
**R⁴** is selected from halo, C₁₋₄alkoxy or C₁₋₄alkylS(O)ₐ wherein a is 0 to 2; wherein that **R⁴** may be optionally substituted on carbon by one or more **R¹⁶;** wherein **R¹⁶** is independently selected from hydroxy and *N*,*N*-(C₁₋₄alkyl)₂amino;
**R⁵** is a group of formula **(IA);**
**Ring A** is aryl or heteroaryl; wherein **Ring A** is optionally substituted by one or more substituents selected from **R¹⁷;**
**R⁷** is hydrogen, C₁₋₄alkyl or carbocyclyl;
**R⁸** is hydrogen or methyl;
**R⁹** is hydrogen or methyl;
**R¹¹** is carboxy, -P(O)(OH)(OR^{c}) or a group of formula **(IB)** (as depicted above);
X is -NH- or -NHC(O)-;
**R¹²** is hydrogen or methyl;
**R¹³** is hydrogen, C₁₋₄alkyl or carbocyclyl; wherein **R¹³** is optionally substituted by one or more substituents selected from **R²⁰;**
**R¹⁴** is hydrogen;
**R¹⁵** is carboxy or sulpho;
**R¹⁷** is selected from halo, hydroxy, C₁₋₄alkyl or C₁₋₄alkoxy; wherein **R¹⁷** may be optionally substituted on carbon by one or more **R²¹;**
**R²⁰** is hydroxy, carboxy, carbocyclyl or amino; wherein **R²⁰** may be optionally substituted on carbon by one or more **R²²;**
**R²¹** is selected from halo;
**R²²** is hydroxy;
**p** is 1-3; wherein the values of **R¹³** may be the same or different.
**q** is 0-1;
**r** is 0-3; wherein the values of **R¹⁴** may be the same or different; and wherein if **q** is 1, **r** is not 0;
**m** is 0-2; and
**n** is 1-3;
or a pharmaceutically acceptable salt thereof.

Therefore in another additional further aspect, there is provided a compound of formula **(I)** as depicted above wherein:
**R¹** and **R²** are independently selected from C₁₋₄alkyl;
**R^{x}** and **R^{y}** are both hydrogen;
**R^{z}** is selected from halo, amino, C₁₋₆alkyl, C₁₋₆alkoxycarbonylamino or *N'*-(C₁₋₆alkyl)ureido; **v** is 0 or 1;
**R³** and **R⁶** are hydrogen;
one of **R⁴** and **R⁵** is a group of formula **(IA)** (as depicted above) and the other is selected from hydrogen, halo, C₁₋₄alkoxy or C₁₋₄alkylS(O)ₐ wherein a is 0 to 2; wherein that **R⁴** or **R⁵** may be optionally substituted on carbon by one or more **R¹⁶;** wherein **R¹⁶** is independently selected from hydroxy, carboxy and *N*,*N*-(C₁₋₄alkyl)2amino;
**X** is -0-;
**R⁷** is hydrogen, methyl or phenyl;
**R⁸** is hydrogen or methyl;
**Ring A** is aryl or heteroaryl; wherein **Ring A** is optionally substituted by one or more substituents selected from **R¹⁷**; wherein **R¹⁷** is selected from halo, hydroxy, C₁₋₄alkyl or C₁₋₄alkoxy; wherein **R¹⁷** may be optionally substituted on carbon by one or more R**²¹;** wherein **R²¹** is selected from halo;
**R⁹** is hydrogen or methyl;
**R¹⁰** is hydrogen;
**R¹¹** is carboxy, -P(O)(OH)(OR^{c}) wherein R^{c} is selected from C₁₋₄alkyl or a group of formula **(IB)** (as depicted above);
**R¹²** is hydrogen or methyl;
**Y** is -NH- or -NHC(O)-;
**R¹³** is hydrogen, C₁₋₄alkyl, carbocyclyl or **R²³;** wherein **R¹³** is optionally substituted by one or more substituents selected from **R²⁰;** wherein **R²⁰** is hydroxy, C₁₋₄alkylS(O)ₐ wherein a is 0, C₁₋₄alkoxy, amino, carbocyclyl, heterocyclyl or mercapto; wherein **R²⁰** may be independently optionally substituted on carbon by one or more **R²²; R²²** is selected from hydroxy; and **R²³** is carboxy;
**R¹⁴** is selected from hydrogen, C₁₋₄alkyl or carbocyclyl; wherein said C₁₋₄alkyl or carbocyclyl may be optionally substituted by one or more substituents selected from **R²⁰;** and **R²⁰** is hydroxy;
**R¹⁵** is carboxy, sulpho, phosphono, -P(O)(OR^{e})(OR^{f}), -P(O)(OH)(OR^{e}), -P(O)(OH)(R^{e}) or -P(O)(OR^{e})(R^{f}) wherein R^{e} and R^{f} are independently selected from C₁₋₄alkyl or **R¹⁵** is a group of formula **(IC)** (as depicted above);
**R²⁴** is hydrogen;
**R²⁵** is hydrogen;
**R²⁶** is carboxy;
**p** is 1-3; wherein the values of **R¹³** may be the same or different;
**q** is 0-1;
**r** is 0-3; wherein the values of **R¹⁴** may be the same or different;
**m** is 0-2; wherein the values of **R¹⁰** may be the same or different;
**n** is 1-2; wherein the values of **R⁷** may be the same or different;
**z** is 0-1; wherein the values of **R²⁵** may be the same or different;
or a pharmaceutically acceptable salt thereof.
In another aspect, preferred compounds are any one of the Examples or a pharmaceutically acceptable salt thereof.

Further disclosed herein is a combination comprising
(i) a compound of formula II 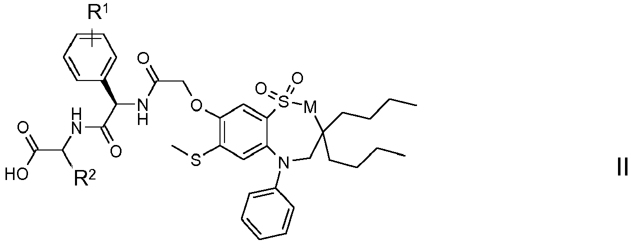 wherein
   **M** is -CH₂ or NH;
   **R¹** is H or OH; and
   **R²** is H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, CH₂OCH₃, -CH(OH)CH₃, -CH₂SCH₃, or -CH₂CH₂-S-CH₃; or a pharmaceutically acceptable salt thereof; and
(ii) at least one other active substance selected from an IBAT inhibitor; an enteroendocrine peptide or enhancer thereof; a dipeptidyl peptidase-IV inhibitor; a biguanidine; an incretin mimetic; a thiazolidinone; a PPAR agonist; a HMG Co-A reductase inhibitor; a bile acid binder; and a TGR5 receptor modulator; or a pharmaceutically acceptable salt of any one of said active substances;
   wherein the compound of formula (II) and the at least one other active substance is administered simultaneously, sequentially or separately.

Further disclosed herein is a combination comprising a compound of formula (II) selected from the group consisting of:
1,1-Dioxo-3,3-dibutyl-5-phenyl-7-methylthio-8-(*N*-{(R)-α-[*N*-(carboxymethyl)carbamoyl] benzyl} carbamoylmethoxy)-2,3,4,5-tetrahydro-1. 2. 5-benzothiadiazepine;
1,1-Dioxo-3,3-dibutyl-5-phenyl-7-methylthio-8-(*N*-{(R)-α-[*N*'-((S)-1-carboxyethyl) carbamoyl] benzyl} carbamoylmethoxy)-2, 3,4, 5-tetrahydro-1,5-benzothiazepine;
1,1-Dioxo-3,3-dibutyl-5-phenyl-7-methylthio-8-(*N*-{(R)-α-[*N*-((S)-1-carboxypropyl) carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthioethyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxybutyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxyethyl) carbamoyl]benzyl}carbamoylmethoxy)-2,3,4,5-tetrahydro-1,2,5-benzothiadiazepine;
1,1-Dioxo-3, 3-dibutyl-5-phenyl-7-methylthio-8-(*N*-{(R)-α-[*N*'-((S)-1-carboxypropyl) carbamoyl]-4-hydroxybenzyl} carbamoylmethoxy) -2,3,4, 5-tetrahydro-1, 5-benzothiazepine;
1,1-Dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-1'-phenyl-1'-[*N'*-(carboxymethyl) carbamoyl] methyl} carbamoylmethoxy)-2,3,4,5-tetrahydro-1,5-benzothiazepine;
or a pharmaceutically acceptable salt of any such compound; and
(ii) at least one other active substance selected from an IBAT inhibitor; an enteroendocrine peptide or enhancer thereof; a dipeptidyl peptidase-IV inhibitor; a biguanidine; an incretin mimetic; a thiazolidinone; a PPAR agonist; a HMG Co-A reductase inhibitor; a bile acid binder; and a TGR5 receptor modulator; or a pharmaceutically acceptable salt of any one of said active substances;
wherein the compound of formula (II) and the at least one other active substance is administered simultaneously, sequentially or separately.

In a first aspect, the invention relates to a combination comprising an IBAT inhibitor and a bile acid binder for simultaneous, sequential or separate administration, wherein the bile acid binder is formulated for colon release, for use in the treatment of a liver disease selected from the group consisting of primary biliary cirrhosis (PBC); progressive familial intrahepatic cholestasis (PFIC); Alagilles syndrome (ALGS); primary sclerosing cholangitis (PSC); non-alcoholic steatohepatitis (NASH); and pruritus of cholestatic liver disease; wherein the IBAT inhibitor is a compound of formula II 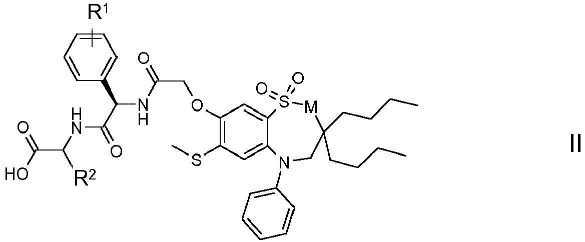 wherein
**M** is -CH₂ or NH;
**R¹** is H or OH; and
**R²** is H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, CH₂OCH₃, -CH(OH)CH₃, -CH₂SCH₃, or -CH₂CH₂-S-CH₃;
or a pharmaceutically acceptable salt thereof.

In a preferred aspect, the compound of formula (II) is selected from the group consisting of:
1,1-Dioxo-3,3-dibutyl-5-phenyl-7-methylthio-8-(*N*-{(R)-α-[*N*-(carboxymethyl)carbamoyl] benzyl} carbamoylmethoxy)-2,3,4,5-tetrahydro-1. 2. 5-benzothiadiazepine;
1,1-Dioxo-3,3-dibutyl-5-phenyl-7-methylthio-8-(*N*-{(R)-α-[*N'*-((S)-1-carboxyethyl) carbamoyl] benzyl} carbamoylmethoxy)-2, 3,4, 5-tetrahydro-1,5-benzothiazepine;
1,1-Dioxo-3,3-dibutyl-5-phenyl-7-methylthio-8-(*N*-{(R)-α-[*N*-((S)-1-carboxypropyl) carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthioethyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxybutyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxyethyl) carbamoyl]benzyl}carbamoylmethoxy)-2,3,4,5-tetrahydro-1,2,5-benzothiadiazepine;
1,1-Dioxo-3, 3-dibutyl-5-phenyl-7-methylthio-8-(*N*-{(R)-α-[*N*'-((S)-1-carboxypropyl) carbamoyl]-4-hydroxybenzyl} carbamoylmethoxy) -2,3,4, 5-tetrahydro-1, 5-benzothiazepine;
1,1-Dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-1'-phenyl-1'-[*N'*-(carboxymethyl) carbamoyl] methyl} carbamoylmethoxy)-2,3,4,5-tetrahydro-1,5-benzothiazepine;
or a pharmaceutically acceptable salt thereof.

Compounds of the formula (II) may have chiral centres and/or geometric isomeric centres (E-and Z-isomers), and it is to be understood that the invention encompasses all such optical, diastereoisomers and geometric isomers that possess IBAT inhibitory activity.

The invention relates to any and all tautomeric forms of the compounds of the formula (II) that possess IBAT inhibitory activity.

The invention also relates all possible isomers of the compounds of the invention such as optical and/or geometrical, pure or as a mixture, in all proportions, of the said compounds of formulas I and I and those specifically mentioned and the possible tautomeric forms.

In certain embodiments, compounds described herein have one or more chiral centres. As such, all stereoisomers are envisioned herein. In various embodiments, compounds described herein are present in optically active or racemic forms. It is to be understood that the compounds of the present invention encompasses racemic, optically-active, regioisomeric and stereoisomeric forms, or combinations thereof that possess the therapeutically useful properties described herein. Preparation of optically active forms is achieve in any suitable manner, including by way of example, by resolution of the racemic form by recrystallization techniques, by synthesis from optically-active starting materials, by chiral synthesis, or by chromatographic separation using a chiral stationary phase. In some embodiments, mixtures of one or more isomer is utilized as the therapeutic compound described herein. In certain embodiments, compounds described herein contains one or more chiral centres. These compounds are prepared by any means, including enantioselective synthesis and/or separation of a mixture of enantiomers and/or diastereomers. Resolution of compounds and isomers thereof is achieved by any means including, by way of example, chemical processes, enzymatic processes, fractional crystallization, distillation, chromatography, and the like.

### Compounds for use in combination with an IBAT inhibitor compound of the invention.

### Bile acid binders (bile acid sequestrants, resins)

### The following bile acid binders may be used according to the invention

Cholestyramine, a hydrophilic polyacrylic quaternary ammonium anion exchange resin, which is known to be effective in reducing blood cholesterol levels. Cholestyramine, and various compositions including cholestyramine, are described, for example, in British Pat Nos. 929,391 and 1,286, 949; and U. S. Patent Nos. 3,383, 281; 3,308, 020; 3,769, 399; 3,846, 541; 3,974, 272; 4,172, 120; 4,252, 790; 4,340, 585; 4,814, 354; 4,874, 744; 4,895, 723; 5,695, 749; and 6,066, 336. Cholestyramine is commercially available from Novopharm, USA Inc (Questrans Light), Upsher-Smith (PREVALITE (D) and Apothecon. As used herein, "cholestyramine" includes any such composition comprising cholestyramine, or pharmaceutically acceptable salts thereof. Questrans™

Questran Light Questrans Light (cholestyramine) is a non-absorbable anion binding resin FDA approved for the treatment of hypercholesterolemia.
An amine polymer having a first substituent, bound to a first amine of the amine polymer, that includes a hydrophobic aliphatic moiety, and a second substituent, bound to a second amine of the amine polymer, that includes an aliphatic quaternary amine-containing moiety as described in USP 5,693,675 and 5,607,669.

The salt of an alkylated and cross linked polymer comprising the reaction product of:(a) one or more cross linked polymers, or salts and copolymers thereof having a repeat unit selected from the group consisting of: (NR-CH₂CH₂)n (2) and (NR-CH₂CH₂-NR-CH₂CH₂-NR-CH₂CHOH-CH₂)n (3) where n is a positive integer and each R, independently, is H or a C1-C8 alkyl group;(b) at least one aliphatic alkylating agent, said reaction product characterized in that:(i) at least some of the nitrogen atoms in said repeat units unreacted with said alkylating agent;(ii) less than 10 mol percent of the nitrogen atoms in said repeat units reacting with said alkylating agent forming quaternary ammonium units; and(iii) a fixed positive charge and one or more counter ions, such as Colesevelam and colesevelam hydrochloride

Suitable bile acid binders for such a combination therapy are resins, such as cholestyramine and cholestipol. One advantage is that the dose of bile acid binder might be kept lower than the therapeutic dose for treatment of cholesterolemia in single treatment comprising solely a bile acid binder. By a low dose of bile acid binder any possible side effects caused by poor tolerance of the patient to the therapeutic dose could also be avoided.

Another useful bile acid binder is a water insoluble non-toxic polymeric amine having a molecular weight in excess of 3,000, having the property of binding at least 30% of the available glycocholic acid within 5 minutes when exposed to an aqueous solution of an equal weight of said acid, having a polymer skeleton inert to digestive enzymes, and having a water content greater than 65% after equilibration with air at 100% relative humidity, egg, cholestipol described in USP 3,383,281,

In a further aspect of the invention a suitable bile acid binder is one of cholestyramine, cholestipol or colesevelam.

A preferred aspect of the present invention is the use of colesevelam as the bile acid binder.

### Other active compounds for use in combination with an IBAT inhibitor compound of the invention.

Further disclosed herein is the administration of a combination comprising an effective amount of a IBAT inhibitor compound or a pharmaceutically acceptable salt thereof, and a bile acid binder, wherein the formulation is designed to deliver the bile acid binder in the colon, with the simultaneous, sequential or separate administration one or more of the following agents selected from:

### Statins

In another aspect, an IBAT inhibitor compound e.g. a compound of formula (I) or (II) or a pharmaceutically acceptable salt thereof, may be administered in association with an HMG Co-A reductase inhibitor, or pharmaceutically acceptable salts thereof. Suitable HMG Co-A reductase inhibitors, pharmaceutically acceptable salts thereof are statins well known in the art. Particular statins are fluvastatin, lovastatin, pravastatin, simvastatin, atorvastatin, cerivastatin, bervastatin, dalvastatin, mevastatin and (E)-7- [4- (4-fluorophenyl)-6-isopropyl-2- [methyl (methylsulphonyl) amino] pyrimidin-5-yl] (3R, 5S)-3,5-dihydroxyhept-6-enoic acid (rosuvastatin), or a pharmaceutically acceptable salt thereof. A particular statin is atorvastatin, or a pharmaceutically acceptable salt thereof. A more particular statin is atorvastatin calcium salt. A further particular statin is (E)-7- [4- (4-fluorophenyl)-6-isopropyl-2- [methyl (methylsulphonyl) amino] pyrimidin-5-yl] (3R, 5S)-3,5-dihydroxyhept-6-enoic acid (rosuvastatin), or a pharmaceutically acceptable salt thereof. Other particular statins are rosuvastatin calcium salt and pitavastatin, (HMG CoA reductase inhibitor).

In an additional aspect, the compound of formula (I), or a pharmaceutically acceptable salt thereof may be administered in association with an HMG Co-A reductase inhibitor, or a pharmaceutically acceptable salt thereof, and/or a bile acid binder thereby avoiding a possible risk of excess of bile acids in colon caused by the inhibition of the ileal bile acid transport system. An excess of bile acids in the visceral contents may cause diarrhoea. An HMG CoA-reductase inhibitor, or a pharmaceutically acceptable salt thereof will by its action decrease the endogenous cholesterol available for the bile acid synthesis and have an additive effect in combination with the compound of formula (I), or a pharmaceutically acceptable salt thereof on lipid lowering.

A CETP (cholesteryl ester transfer protein) inhibitor, for example those referenced and described in WO 00/38725 page 7 line 22-page 10, line 17.

A cholesterol absorption antagonist for example azetidinones such as SCH 58235 and those described in US 5,767,115;
MTP (microsomal transfer protein) inhibitor for example those described in Science, 282,751-54,1998;
A fibric acid derivative; for example clofibrate, gemfibrozil, fenofibrate, ciprofibrate and bezafibrate;
A nicotinic acid derivative, for example, nicotinic acid (niacin), acipimox and niceritrol;
A phytosterol compound for example stanols;
Probucol;
An anti-obesity compound for example orlistat (EP 129,748) and sibutramine (GB 2,184,122 and US 4,929,629);
An antihypertensive compound for example an angiotensin converting enzyme (ACE) inhibitor, an angiotensin II receptor antagonist, an adrenergic blocker, an alpha adrenergic blocker, a beta adrenergic blocker, a mixed alpha/beta adrenergic blocker, an adrenergic stimulant, calcium channel blocker, a diuretic or a vasodilator;
Insulin;
Sulphonylureas including glibenclamide and/or tolbutamide;

### Biguanides

The other active compound may be a biguanide, which may lower blood and/or plasma glucose levels. Examples of biguanides include buformin, metformin, phenformin, proguanil or the like.

Acarbose;
or a pharmaceutically acceptable salt thereof, optionally together with a pharmaceutically acceptable diluent or carrier to a warm-blooded animal, such as man in need of such therapeutic treatment.

### ACE inhibitors

Particular ACE inhibitors or pharmaceutically acceptable salts thereof, including active metabolites, which can be used in combination with a compound of formula (I) include the following compounds: alacepril, alatriopril, altiopril calcium, ancovenin, benazepril, benazepril hydrochloride, benazeprilat, benzoylcaptopril, captopril, captopril-cysteine, captopril- glutathione, ceranapril, ceranopril, ceronapril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, glycopril, hemorphin-4, idrapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, utibapril, zabicipril, zabiciprilat, zofenopril and zofenoprilat. Preferred ACE inhibitors are ramipril, ramiprilat, lisinopril, enalapril and enalaprilat. More preferred ACE inhibitors are ramipril and ramiprilat.

### Angiotensin II antagonists

Preferred angiotensin II antagonists, pharmaceutically acceptable salts thereof for use in combination with a compound of formula (I) include the compounds: candesartan, candesartan cilexetil, losartan, valsartan, irbesartan, tasosartan, telmisartan and eprosartan. Particularly preferred angiotensin II antagonists or pharmaceutically acceptable derivatives thereof are candesartan and candesartan cilexetil.

PPAR alpha and/or gamma and/or delta agonists.

In another aspect, the IBAT inhibitor compound, or a pharmaceutically acceptable salt thereof, may be administered in association with a PPAR alpha and/or gamma agonist, or pharmaceutically acceptable salts thereof. Suitable PPAR alpha and/or gamma agonists, pharmaceutically acceptable salts thereof are well known in the art. These include the compounds described in WO 01/12187, WO 01/12612, WO 99/62870, WO 99/62872, WO 99/62871, WO 98/57941, WO 01/40170, J Med Chem, 1996,39,665, Expert Opinion on Therapeutic Patents, 10 (5), 623-634 (in particular the compounds described in the patent applications listed on page 634) and J Med Chem, 2000,43,527. Particularly a PPAR alpha and/or gamma agonist refers to WY-14643, clofibrate, fenofibrate, bezafibrate, GW 9578, troglitazone, pioglitazone, rosiglitazone, eglitazone, proglitazone, BRL-49634, KRP-297, JTT-501, SB 213068, GW 1929, GW 7845, GW 0207, L-796449, L-165041 and GW 2433.

Particularly a PPAR alpha and/or gamma agonist refers to (S)-2-ethoxy-3- [4- (2- {4-methanesulphonyloxyphenyl} ethoxy) phenyl] propanoic acid and pharmaceutically acceptable salts thereof.

Other useful active substances may be antidiabetics, hypoglycaemic active ingredients, cholesterol absorption inhibitors, PPAR delta agonists, fibrates, MTP inhibitors, bile acid absorption inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, alpha-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-bisphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, beta 3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-beta agonists or amphetamines.

Examples of PPAR delta agonists are GW-501516 (501516, GSK-516, GW-516, GW-1516;a peroxisome proliferator-activated receptor (PPAR)-delta agonist, and several other compounds developed from GW-501516, including GI-262570, GW-0072, GW-7845 and GW-7647.

It is disclosed herein that the IBAT inhibitor may be combined with one or more of Atreleuton, Eprotirome, Losmapimod, Ezetimibe (SCH58235), Bezafibrate, Fenofibrate, Varespladib, Darapladib, Lomitapide, Implitapide, Rosiglitazone, Dalcetrapib, Anacetrapib, Lorcaserin, Dapagliflozin, Canagliflozin, Sergliflozin, ASP-1941, Orlistat, Exenatide, Liraglutide, Taspoglutide, Tulaglutide, Pramlintide, Lixisenatide, Albiglutide, Pioglitazone, Sodelglitazar, Netoglitazone, Indeglitazar, Naveglitazar, Lobeglitazone, Aleglitazar, Bromocriptine, Tesofensine, Alogliptin, Vildagliptin, Saxagliptin, Sitagliptin, Denagliptin, Gemigliptin, Linagliptin, Dutogliptin, Teneligliptin, LC-150444, Laropiprant extended release niacin, Simvastatin ezetimibe, Rosuvastatin fenofibrate, Rosuvastatin ezetimibe and Atorvastatin ezetimibe.

### Combinations with Tredaptive, Vytorin and Certriad may be used

It is disclosed herein that the IBAT inhibitors are combined with at least one other active substance selected from dipeptidyl peptidase-IV-inhibitors, statins, PPAR **γ** agonist, statins and bile acid binders in any combination.

It is disclosed herein that the IBAT inhibitors are combined with at least one DPPIV, at least one PPAR **γ** agonist, such as Sitagliptin and Pioglitazon.

It is disclosed herein that the IBAT inhibitors are combined with at least one statin e.g. Sitagliptin and Simvastatin and at least one DPPIV.

In certain instances, use of the compounds reduces or inhibits recycling of bile acid salts in the gastrointestinal tract. In some embodiments, the bile transport inhibitors are non-systemic compounds. In other embodiments, the bile acid transporter inhibitors are systemic compounds. In certain embodiments, the bile transport inhibitor s described herein enhance L-cell secretion of enteroendocrine peptides. In certain instances, increased L-cell secretion of enteroendocrine peptides is associated with induction of satiety and/or reduction of food intake (caloric intake) and subsequent weight loss. In some embodiments, increased L-cell secretion of enteroendocrine peptides is associated with a reduction in blood and/or plasma glucose levels in a hyperglycaemic individual. In some instances, increased L-cell secretion of enteroendocrine peptides is associated with increased insulin sensitivity.

In some embodiments, contacting the distal ileum of an individual in need thereof with an IBAT inhibitor a. reduces food intake in the individual; b. induces satiety in the individual; c. reduces blood and/or plasma glucose levels in the individual; d. treats a metabolic disorder in the individual; e. reduces the weight of the individual; f. stimulates L-cells in the distal gastrointestinal tract of the individual; g. increases the concentration of bile acids and salts thereof in the vicinity of L-cells in the distal gastrointestinal tract of the individual; h. enhances enteroendocrine peptide secretion in the individual; or i. any combination thereof.

It is disclosed herein that the IBAT inhibitor may be combined with one or more of any of the above mentioned other compounds.

In some embodiments, the IBAT inhibitor is not systemically absorbed. In some other embodiments, the IBAT inhibitor is systemically absorbed.

In some embodiments, the uses described above further comprise administration of a second agent selected from a DPP-IV inhibitor, a biguanide, an incretin mimetic, a thiazolidinedione, GLP-1 or an analog thereof, and a TGR5 agonist. In some embodiments, the second agent is a DPP-IV inhibitor.

In some embodiments, the uses described herein enhance enteroendocrine peptide secretion in an individual in need thereof. In some of such embodiments, the enteroendocrine peptide is GLP-1, GLP-2, PYY, oxyntomodulin, or a combination thereof.

In some embodiments, contacting the distal ileum of an individual in need thereof with an IBAT inhibitor increases the level of GLP-1 in the blood and/or plasma of the individual by from about 2 times to about 6 times the level of GLP-1 in the blood and/or plasma of the individual prior to contacting the distal ileum of the individual with the IBAT inhibitor.

In some embodiments, contacting the distal ileum of an individual in need thereof with an IBAT inhibitor reduces the level of glucose in the blood and/or plasma of the individual by at least 30% compared to the level of glucose in the blood and/or plasma of the individual prior to contacting the distal ileum of the individual with the IBAT inhibitor.

In some embodiments, contacting the distal ileum of an individual in need thereof with an IBAT inhibitor maintains reduced blood and/or plasma glucose levels in the individual for at least 24 hours compared to blood and/or plasma glucose levels in the individual prior to contacting the distal ileum of the individual with the IBAT inhibitor.

In some embodiments, the IBAT inhibitor is administered orally. In some embodiments, the IBAT inhibitor is administered as an ileal-pH sensitive release formulation that delivers the IBAT inhibitor to the distal ileum and/or colon and/or rectum of an individual. In some embodiments, the IBAT inhibitor is administered as an enterically coated formulation.

In some embodiments of the uses described above, the IBAT inhibitor is a compound of Formula I as described herein. In some embodiments of the uses described above, the IBAT inhibitor is a compound of Formula II as described herein.

In some embodiments of the uses described above, the IBAT inhibitor is administered before ingestion of food. In some embodiments of the uses described above, the IBAT inhibitor is administered less than about 60 minutes before ingestion of food. In some embodiments of the uses described above, the IBAT inhibitor is administered less than about 30 minutes before ingestion of food. In some embodiments of the uses described above, the IBAT inhibitor is administered after ingestion of food.

All substances mentioned herein including both IBAT inhibitors and other active substances may also be used in the form of pharmaceutically acceptable salts and esters where applicable.

### Incretines and hormones produced by the L cells

It is disclosed herein that the other active substances may be Incretines and/or hormones produced by the L cells.

In some embodiments, the additional therapeutic agent is an L-cell endocrine peptide enhancer. In some instances, the L-cell endocrine peptide enhancer is a GLP-1 enhancer. In some embodiments, the GLP-1 enhancer is GLP-1, a GLP-1 secretion enhancer, a GLP-1 degradation inhibitor, the like, or a combination thereof. In certain instances, enhanced GLP-1 concentration provides a reduction in food intake and/or a reduction in gastric emptying in human subjects.

In some embodiments, the L-cell endocrine peptide enhancer is a GLP-2 enhancer. In certain instances, the GLP-2 enhancer is GLP-2, a GLP-2 secretion enhancer, a GLP-2 degradation inhibitor, the like, or a combination thereof. In certain instances, enhanced GLP-2 secretion inhibits gastric emptying and reduces intestinal permeability. In some instances, enhanced GLP-2 secretion inhibits gastric acid secretion. In some instances, enhanced GLP-2 secretion reduces or prevents inflammation in the gastrointestinal tract (gastrointestinal enteritis). In some instances, enhanced GLP-2 secretion regenerates and/or heals injury to gastrointestinal tissues (e.g., radiation enteritis).

In some instances, the L-cell endocrine peptide enhancer is a PYY enhancer. In some instances, enhanced secretion of PYY provides a reduction in sensation of hunger. In some instances, the L-cell endocrine peptide enhancer is an oxyntomodulin enhancer. In some instances, the enhanced secretion of oxyntomodulin inhibits meal-stimulated gastric secretion.

### Incretin Mimetics

In some embodiments, the additional therapeutic agent is an incretin mimetic. In some embodiments, an incretic mimic augments pancreas response to ingestion of food. In some instances, administration of an incretin mimetic in combination with any of the compounds described herein lowers blood and/or plasma glucose levels. Examples of incretin mimetics include exenatide (Byetta.RTM.).

One currently used therapy for the treatment is a subcutaneous injection of exenatide (Byetta.RTM.). In some embodiments, an oral combination of an IBAT inhibitor and a DPP-IV inhibitor is equally or more effective than an injection of exenatide in reducing plasma glucose levels. In some embodiments, an oral combination of an IBAT inhibitor and a DPP-IV inhibitor reduces or eliminates discomfort associated with injections of glucose-lowering medications.

### Enteroendocrine Peptides

In some embodiments, the additional therapeutic agent is an enteroendocrine peptide. In some embodiments, enteroendocrine peptides reverse insulin resistance and lower blood and/or plasma glucose levels. Examples of enteroendocrine peptides that are administered as additional therapeutic agents include GLP-1 or GLP-1 analogs such as Taspoglutide.RTM. (Ipsen), or the like.

### Combination Therapy with IBAT inhibitor and DPP-IV Inhibitor

In specific embodiments, the additional therapeutic agent inhibits degradation of L-cell enteroendocrine peptides. In certain embodiments, the additional therapeutic agent is a DPP-IV inhibitor. In certain instances, administration of an IBAT inhibitor to an individual in need thereof enhances the secretion of GLP-1; administration of a DPP-IV inhibitor in combination with the IBAT inhibitor reduces or inhibits degradation of GLP-1 thereby prolonging the therapeutic benefit of enhanced levels of GLP-1. In some embodiments, administration of an IBAT inhibitor reduces weight of an individual. In some embodiments, administration of an IBAT inhibitor in combination with a DPP-IV inhibitor reduces weight of an individual.

Another use is a combination of metformin and sitagliptin (Janumet.RTM.). At doses of 0, 3, 30, 100, 300 mg/kg doses of metformin in combination with 30 mg/kg of sitagliption, reductions in plasma glucose concentrations are observed from 3 hours till about 6 hours post-dose. In some embodiments, a combination of an IBAT inhibitor and sitagliptin maintains reduced plasma glucose concentrations for a longer duration of time (e.g., at least 24 hours) compared to a combination of metformin and sitagliptin (about 6 hours). In some instances IBAT inhibitor therapy eliminates side effects associated with metformin therapy and/or DPP-IV inhibitor therapy.

DPP-IV inhibitors suitable for use with the uses described herein include (2S)-1-{2-[(3-hydroxy-1-adamantyl)amino]acetyl}pyrrolidine-2-carbonitrile (vildagliptin), (3R)-3-amino-1-[9-(trifluoromethyl)-1,4,7,8-tetrazabicyclo[4.3.0]nona-6,8- -dien-4-yl]-4-(2,4,5-trifluorophenyl)butan-1-one (sitagliptin), (1S,3S,5S)-2-[(2S)-2-amino-2-(3-hydroxy-1-adamantyl)acetyl]-2-azabicyclo[- 3.1.0]hexane-3-carbonitrile (saxagliptin), and 2-({6-[(3R)-3-aminopiperidin-1-yl]-3-methyl-2,4-dioxo-3,4-dihydropyrimidi- n-1 (2H)-yl}methyl)benzonitrile (alogliptin).

In some embodiments of any of the uses described herein, administration of an ASBT inhibitor described herein in combination with a DPP-IV inhibitor increases the level of GLP-1 in the blood and/or plasma of an individual by from about 1.1 times to about 30 times compared to the level of GLP-1 in the blood and/or plasma of the individual prior to administration of the IBAT inhibitor in combination with the DPP-IV inhibitor. In some embodiments of any of the uses described herein, administration of an ASBT inhibitor described herein in combination with a DPP-IV inhibitor increases the level of GLP-1 in the blood and/or plasma of an individual by from about 1.1 times to about 20 times compared to the level of GLP-1 in the blood and/or plasma of the individual prior to administration of the IBAT inhibitor in combination with the DPP-IV inhibitor. In some embodiments of any of the uses described herein, administration of an ASBT inhibitor described herein in combination with a DPP-IV inhibitor increases the level of GLP-1 in the blood and/or plasma of an individual by from about 1.5 times to about 10 times compared to the level of GLP-1 in the blood and/or plasma of the individual prior to administration of the IBAT inhibitor in combination with the DPP-IV inhibitor. In some embodiments of any of the uses described herein, administration of an ASBT inhibitor described herein in combination with a DPP-IV inhibitor increases the level of GLP-1 in the blood and/or plasma of an individual by from about 2 times to about 8 times compared to the level of GLP-1 in the blood and/or plasma of the individual prior to administration of the IBAT inhibitor in combination with the DPP-IV inhibitor. In some embodiments of any of the uses described herein, administration of an ASBT inhibitor described herein in combination with a DPP-IV inhibitor increases the level of GLP-1 in the blood and/or plasma of an individual by from about 2 times to about 6 times compared to the level of GLP-1 in the blood and/or plasma of the individual prior to administration of the IBAT INHIBITOR in combination with the DPP-IV inhibitor. In some instances, an increase in GLP-1 level of from about 2 times to about 3 times following the administration of an ASBT inhibitor described herein in combination with a DPP-IV inhibitor compared to the level of GLP-1 in the blood and/or plasma of the individual prior to administration of the IBAT INHIBITOR in combination with the DPP-IV inhibitor may be used. In some instances, an increase in GLP-1 level of from about 3 times to about 8 times following the administration of an ASBT inhibitor described herein in combination with a DPP-IV inhibitor compared to the level of GLP-1 in the blood and/or plasma of the individual prior to administration of the IBAT inhibitor in combination with a DPP-IV inhibitor may be used.

In certain embodiments of any of the uses described herein, administration of an IBAT inhibitor in combination with a DPP-IV inhibitor reduces blood and/or plasma sugar levels by at least 20%, at least 30%, at least 40%, at least 50% at least 60%, at least 70% or at least 80% compared to blood and/or plasma sugar levels prior to administration of the IBAT inhibitor in combination with a DPP-IV inhibitor. In some embodiments of any of the uses described herein, administration of an IBAT inhibitor in combination with a DPP-IV inhibitor reduces blood and/or plasma sugar levels by at least 20% compared to blood and/or plasma sugar levels prior to administration of the IBAT inhibitor in combination with a DPP-IV inhibitor. In some embodiments of any of the uses described herein, administration of an IBAT inhibitor in combination with a DPP-IV inhibitor reduces blood and/or plasma sugar levels by at least 30% compared to blood and/or plasma sugar levels prior to administration of the IBAT inhibitor in combination with a DPP-IV inhibitor. In some embodiments of any of the uses described herein, administration of an IBAT inhibitor in combination with a DPP-IV inhibitor reduces blood and/or plasma sugar levels by at least 40% compared to blood and/or plasma sugar levels prior to administration of the IBAT INHIBITOR in combination with a DPP-IV inhibitor.

In some embodiments of any of the uses described herein, administration of an IBAT inhibitor in combination with a DPP-IV inhibitor reduces blood and/or plasma sugar levels for a longer period of time (e.g., at least 24 hours) compared to reduction in blood and/or plasma sugar levels upon administration of metformin in combination with a DPP-IV inhibitor. In some embodiments of any of the uses described herein, administration of a single dose of an IBAT inhibitor in combination with a DPP-IV inhibitor sustains reduced blood and/or plasma sugar levels for at least 6 hours, at least 12 hours, at least 14 hours, at least 16 hours, at least 18 hours, at least 20 hours, at least 24 hours, at least 30 hours, at least 36 hours or at least 48 hours compared to reduction in blood and/or plasma sugar levels upon administration of a single dose of metformin in combination with a DPP-IV inhibitor.

In some embodiments of any of the uses described herein, administration of an IBAT inhibitor in combination with a DPP-IV inhibitor results in higher levels of GLP-1 in blood and/or plasma of an individual compared to levels of GLP-1 in blood and/or plasma of a normal individual. In some embodiments of any of the uses described herein, administration of an IBAT inhibitor in combination with a DPP-IV inhibitor results in higher levels of GLP-1 in blood and/or plasma of an individual compared to levels of GLP-1 in blood and/or plasma of an individual undergoing therapy with metformin and/or a DPP-IV inhibitor.

### TGR5 Receptor Modulators

In some instances, the additional therapeutic agent modulates bile acid receptors in the gastrointestinal lumen. In some embodiments, the additional therapeutic agent agonizes or partially agonizes bile acid receptors (e.g., TGR5 receptors or Farnesoid-X receptors) in the gastrointestinal tract. In some embodiments, the additional therapeutic agent is a bile acid analogue. In certain instances the additional therapeutic agent is a TGR5 agonist. In certain instances, administration of a TGR5 agonist in combination with any of the compounds described herein enhances the secretion of enteroendocrine peptides from L-cells. TGR5 modulators (e.g., agonists) include the compounds described in, WO 2008/091540, WO 2008/067219 and U.S. Appl. No. 2008/0221161.

### Thiazolidinediones

In some embodiments, the additional therapeutic agent is a thiazolidinedione. In some instances thiazolidinediones reverse insulin resistance and lower blood and/or plasma glucose levels. Examples of thiazolidinediones include Rosiglitazone (Avandia), Pioglitazone (Actos), Troglitazone (Rezulin), MCC-555, rivoglitazone, ciglitazone or the like.

### Combination Therapy with IBAT I inhibitor, Biliary Shunt and DPP-IV Inhibitor

In some embodiments, an IBAT INHIBITOR is administered in combination with a DPP-IV inhibitor and/or a biliary shunt. Examples of biliary shunts are described in WO 2007/0050628. In some of such embodiments, a biliary shunt moves bile acid to the distal ileum and/or the rectum and/or the colon thereby increasing the concentration of bile acids in the vicinity of L-cells present in the distal portion of the gastrointestinal tract. In some instances such an increase in the concentration of bile acids in the vicinity of L-cells increases the secretion of GLP-1 from L-cells thereby inducing satiety and/or reduction in hunger and/or weight loss and/or reduction in plasma glucose levels or any combination thereof.

An IBAT inhibitor and a second active ingredient are used such that the combination is present in a therapeutically effective amount. That therapeutically effective amount arises from the use of a combination of an IBAT inhibitor and the other active ingredient (e.g., a DPP-IV inhibitor) wherein each is used in a therapeutically effective amount, or by virtue of additive or synergistic effects arising from the combined use, each can also be used in a subclinical therapeutically effective amount, i.e., an amount that, if used alone, provides for reduced effectiveness for the therapeutic purposes noted herein, provided that the combined use is therapeutically effective. In some embodiments, the use of a combination of an IBAT inhibitor and any other active ingredient as described herein encompasses combinations where the IBAT inhibitor or the other active ingredient is present in a therapeutically effective amount, and the other is present in a subclinical therapeutically effective amount, provided that the combined use is therapeutically effective owing to their additive or synergistic effects. As used herein, the term "additive effect" describes the combined effect of two (or more) pharmaceutically active agents that is equal to the sum of the effect of each agent given alone. A synergistic effect is one in which the combined effect of two (or more) pharmaceutically active agents is greater than the sum of the effect of each agent given alone. Any suitable combination of an ASBIT with one or more of the aforementioned other active ingredients and optionally with one or more other pharmacologically active substances is contemplated as being within the scope of the uses described herein.

In some embodiments, the particular choice of compounds depends upon the diagnosis of the attending physicians and their judgment of the condition of the individual and the appropriate treatment protocol. The compounds are optionally administered concurrently (e.g., simultaneously, essentially simultaneously or within the same treatment protocol) or sequentially, depending upon the nature of the disease, disorder, or condition, the condition of the individual, and the actual choice of compounds used. In certain instances, the determination of the order of administration, and the number of repetitions of administration of each therapeutic agent during a treatment protocol, is based on an evaluation of the disease being treated and the condition of the individual.

In some embodiments, therapeutically-effective dosages vary when the drugs are used in treatment combinations. Methods for experimentally determining therapeutically-effective dosages of drugs and other agents for use in combination treatment regimens are described in the literature.

In some embodiments of the combination therapies described herein, dosages of the co-administered compounds vary depending on the type of co-drug employed, on the specific drug employed, on the disease or condition being treated and so forth. In addition, when co-administered with one or more biologically active agents, the compound provided herein is optionally administered either simultaneously with the biologically active agent(s), or sequentially. In certain instances, if administered sequentially, the attending physician will decide on the appropriate sequence of therapeutic compound described herein in combination with the additional therapeutic agent.

The multiple therapeutic agents (at least one of which is a therapeutic compound described herein) are optionally administered in any order or even simultaneously. If simultaneously, the multiple therapeutic agents are optionally provided in a single, unified form, or in multiple forms (by way of example only, either as a single pill or as two separate pills). In certain instances, one of the therapeutic agents is optionally given in multiple doses. In other instances, both are optionally given as multiple doses. If not simultaneous, the timing between the multiple doses is any suitable timing, e.g. from more than zero weeks to less than four weeks. In addition, the combinations, compositions and formulations are not to be limited to the use of only two agents; the use of multiple therapeutic combinations is also envisioned (including two or more compounds described herein).

In certain embodiments, a dosage regimen to treat, prevent, or ameliorate the condition(s) for which relief is sought, is modified in accordance with a variety of factors. These factors include the disorder, from which the subject suffers, as well as the age, weight, sex, diet, and medical condition of the subject. Thus, in various embodiments, the dosage regimen actually employed varies and deviates from the dosage regimens set forth herein.

In some embodiments, the pharmaceutical agents which make up the combination therapy described herein are provided in a combined dosage form or in separate dosage forms intended for substantially simultaneous administration. In certain embodiments, the pharmaceutical agents that make up the combination therapy are administered sequentially, with either therapeutic compound being administered by a regimen calling for two-step administration. In some embodiments, two-step administration regimen calls for sequential administration of the active agents or spaced-apart administration of the separate active agents. In certain embodiments, the time period between the multiple administration steps varies, by way of example, from a few minutes to several hours, depending upon the properties of each pharmaceutical agent, such as potency, solubility, bioavailability, plasma half-life and kinetic profile of the pharmaceutical agent.

### Diseases

Disclosed herein is a combination as herein described, for use in the prophylactic or therapeutic treatment of a liver disease. Examples of liver diseases where a combination as herein described may be useful include liver parenchyma; an Inherited metabolic disorder of the liver; Byler syndrome; a primary defect of bile acid (BA) synthesis such as cerebrotendinous, or xanthomatosis; a secondary defect such as Zellweger*'*s syndrome, neonatal hepatitis, cystic fibrosis, manifestations in the liver, ALGS (Alagilles syndrome), PFIC (progressive familial intrahepatic cholestasis, autoimmune hepatitis, primary biliary cirrhosis (PBC), liver fibrosis, non alcoholic fatty liver disease, NAFLD/NASH, portal hypertension, general cholestasis such as in jaundice due to drugs or during pregnancy, intra and extrahepatic cholestasis such as hereditary forms of cholestasis such as PFIC1, Primary sclerosing cholangitis, gall stones and choledocholithiasis, malignancy causing obstruction of the biliary tree, symptoms (scratching, pruritus) due to cholestasis/jaundice, pancreatitis, chronic autoimmune liver disease leading to progressive cholestasis, or pruritus of cholestatic liver disease; a hepatic disorder or a hepatic related condition, fatty liver, hepatic steatosis, non-alcoholic steatohepatitis (NASH), alcoholic hepatitis, acute fatty liver, fatty liver of pregnancy, drug-induced hepatitis, iron overload disorders, hepatic fibrosis, hepatic cirrhosis, hepatoma, viral hepatitis and problems in relation to tumours and neoplasmas of the liver and of the biliary tract.

### Medicinal and pharmaceutical use of the invention.

Disclosed herein is an oral pharmaceutical formulation comprising an IBAT inhibitor compound or a pharmaceutically acceptable salt thereof and a bile acid binder, wherein the formulation is designed to deliver the bile acid binder in the colon for use in the production of an IBAT inhibitory effect in a warm-blooded animal, such as man.

Also disclosed herein is an oral pharmaceutical formulation comprising an IBAT inhibitor compound or a pharmaceutically acceptable salt thereof and a bile acid binder, and at least one of the above mentioned other active compounds and a bile acid binder of the invention wherein the formulation is designed to deliver the bile acid binder in the colon for use in the production of an IBAT inhibitory effect in a warm-blooded animal, such as man.

Also disclosed herein is an oral pharmaceutical formulation comprising an IBAT inhibitor compound or a pharmaceutically acceptable salt thereof and a bile acid binder, wherein the formulation is designed to deliver the bile acid binder in the colon for use in prophylaxis or treatment of any of the herein mentioned medical indications in a warm-blooded animal, such as man.

Also disclosed herein is an oral pharmaceutical formulation comprising an IBAT inhibitor compound or a pharmaceutically acceptable salt thereof and a bile acid binder, and at least one of the above mentioned other active compounds and a bile acid binder of the invention wherein the formulation is designed to deliver the bile acid binder in the colon for use in prophylaxis or treatment of any of the herein mentioned medical indications in a warm-blooded animal, such as man.

Also disclosed herein is an oral pharmaceutical formulation comprising an IBAT inhibitor compound or a pharmaceutically acceptable salt thereof and a bile acid binder, wherein the formulation is designed to deliver the bile acid binder in the colon for use in the preparation of a pharmaceutical for use in prophylaxis or treatment of any of the herein mentioned medical indications in a warm-blooded animal, such as man.

Also disclosed herein is an oral pharmaceutical formulation comprising an IBAT inhibitor compound or a pharmaceutically acceptable salt thereof and a bile acid binder, and at least one of the above mentioned other active compounds and a bile acid binder of the invention wherein the formulation is designed to deliver the bile acid binder in the colon for use in the preparation of a pharmaceutical for use in prophylaxis or treatment of any of the herein mentioned medical indications in a warm-blooded animal, such as man.
Also disclosed herein is an effective amount of an IBAT inhibitor compound or a pharmaceutically acceptable salt thereof in simultaneous, sequential or separate administration with an effective amount of a bile acid binder, for use in the treatment of any of the herein mentioned medical conditions.

Also disclosed herein is an effective amount of an IBAT inhibitor compound or a pharmaceutically acceptable salt thereof and at least one of the above mentioned other active compounds in simultaneous, sequential or separate administration with an effective amount of a bile acid binder, for use in the treatment of any of the herein mentioned medical conditions.

### Pharmaceutical formulations

Pharmaceutical compositions may be formulated in a conventional manner using one or more physiologically acceptable carriers including, e.g., excipients and auxiliaries which facilitate processing of the active compounds into preparations which are suitable for pharmaceutical use. In certain embodiments, proper formulation is dependent upon the route of administration chosen. A summary of pharmaceutical compositions described herein is found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pa. 1975; Liberman, H. A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

A pharmaceutical composition, as used herein, refers to a mixture of a compound described herein, such as, for example, a compound of Formula I and II and possibly also other active compounds mentioned herein, with other chemical components, such as carriers, stabilizers, diluents, dispersing agents, suspending agents, thickening agents, and/or other excipients. In certain instances, the pharmaceutical composition facilitates administration of the compound to an individual or cell. In certain embodiments of practicing the use provided herein, therapeutically effective amounts of compounds described herein are administered in a pharmaceutical composition to an individual having a disease, disorder, or condition to be treated. In specific embodiments, the individual is a human. As discussed herein, the compounds described herein are either utilized singly or in combination with one or more additional therapeutic agents.

In certain embodiments, the pharmaceutical formulations described herein are administered to an individual in any manner, including one or more of multiple administration routes, such as, by way of example, oral or buccal administration routes.

In certain embodiments, a pharmaceutical compositions described herein includes one or more compound described herein as an active ingredient in free-acid or free-base form, or in a pharmaceutically acceptable salt form. In some embodiments, the compounds described herein are utilized as an N-oxide. In some situations, a compound described herein exists as tautomers. All tautomers are included within the scope of the compounds presented herein. In certain embodiments, a compound described herein exists in an unsolvated or solvated form, wherein solvated forms comprise any pharmaceutically acceptable solvent, e.g., water, ethanol, and the like.

According to the invention, the IBAT inhibitor of formula II and the bile acid binder may be administered simultaneously, separately or sequentially. According to the invention, the IBAT inhibitor and the bile acid binder may be formulated in separate formulations with the IBAT inhibitor formulation releasing the drug immediately or delayed in the distal jejunum or the proximal ileum and the bile acid binder formulation releasing the drug in the colon.

According to the invention, the IBAT inhibitor of formula II and the bile acid binder may be combined in one formulation with the bile acid binder in the core and formulated for release in the colon and the IBAT inhibitor in an outer layer formulated for immediate release or for delayed release in the distal jejunum or the proximal ileum.

According to one embodiment there is provided a novel dosage regime by administrating an IBAT inhibitor of the invention once a day and a bile acid binder once, twice or three times a day.

According to one embodiment the IBAT inhibitor and the bile acid binder are administrated together once, twice or three times a day.

According to one embodiment the IBAT inhibitor is administrated in a formulation releasing it in the small intestine.

According to one embodiment the bile acid binder is administrated in a formulation releasing it in the colon.

An aspect of the invention is a pharmaceutical formulation comprising an IBAT inhibitor of formula II and a bile acid binder, wherein the bile acid binder is in the core and formulated for release in the colon and wherein the IBAT inhibitor is in an outer layer formulated for immediate release or for delayed release in the distal jejunum or the proximal ileum, for use in the treatment of a liver disease selected from the group consisting of primary biliary cirrhosis (PBC); progressive familial intrahepatic cholestasis (PFIC); Alagilles syndrome (ALGS); primary sclerosing cholangitis (PSC); non-alcoholic steatohepatitis (NASH); and pruritus of cholestatic liver disease;
wherein the IBAT inhibitor is a compound of formula II 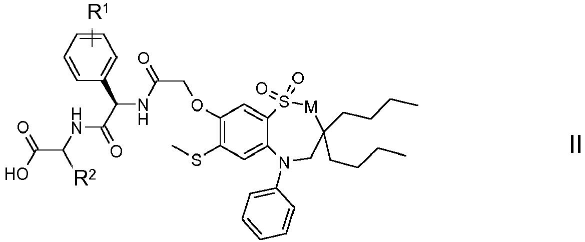 wherein
**M** is -CH₂ or NH;
**R¹** is H or OH; and
**R²** is H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, CH₂OCH₃, -CH(OH)CH₃, -CH₂SCH₃, or -CH₂CH₂-S-CH₃;
or a pharmaceutically acceptable salt thereof.

An aspect of the present invention is a pharmaceutical combination formulation, comprising
(i) an inner core comprising a bile acid binder;
(ii) a colon release layer onto the core;
(iii) an I BAT inhibitor layer onto said colon release layer; and
(iv) an outer protective coating

In this aspect of the invention, the IBAT inhibitor layer comprises the compound of formula II.

In the dosage regime or the formulations mentioned above, the acid binder may be colesvelam and the IBAT inhibitor a compound with formula II or the 14 Examples below.

Dosage forms with different release regimens can be constructed in different ways for instance as described in the following.

### Release in distal jejunum or ileum and/or colon

In certain embodiments, a dosage form comprises a matrix (e.g., a matrix comprising hypermellose) that allows for controlled release of an active agent in the distal jejunum, proximal ileum, distal ileum and/or the colon. In some embodiments, a dosage form comprises a polymer that is pH sensitive (e.g., a MMX.™. matrix from Cosmo Pharmaceuticals) and allows for controlled release of an active agent in the ileum and/or the colon. Examples of such pH sensitive polymers suitable for controlled release include polyacrylic polymers (e.g., anionic polymers of methacrylic acid and/or methacrylic acid esters, e.g., Carbopol.R™. polymers) that comprise acidic groups (e.g., -COOH, -SO₃H) and swell in basic pH of the intestine (e.g., pH of about 7 to about 8). In some embodiments, a dosage form suitable for controlled release in the distal ileum comprises microparticulate active agent (e.g., micronized active agent). In some embodiments, a non-enzymatically degrading poly(dl-lactide-co-glycolide) (PLGA) core is suitable for delivery of an IBAT inhibitor to the distal ileum. In some embodiments, a dosage form comprising an IBAT inhibitor is coated with an enteric polymer (e.g., Eudragit.RTM. S-100 (CAS number: 25086 - 15 - 1), cellulose acetate phthalate, polyvinylacetate phthalate, hydroxypropylmethylcellulose phthalate, anionic polymers of methacrylic acid, methacrylic acid esters or the like) for site specific delivery to the ileum and/or the colon. In some embodiments, bacterially activated systems are suitable for targeted delivery to the ileum.

Examples of micro-flora activated systems include dosage forms comprising amylose, pectin, galactomannan, and/or azo hydrogels and/or glycoside conjugates (e.g., conjugates of D-galactoside, .beta.-D-xylopyranoside or the like) of the active agent. Examples of gastrointestinal micro-flora enzymes include bacterial glycosidases such as, for example, D-galactosidase, beta-D-glucosidase, alpha-L-arabinofuranosidase, beta-D-xylopyranosidase or the like.

A controlled lag time can be triggered by pH changes, redox potential differences or luminal metabolic changes in the gastro-intestinal tract as described in Aliment Pharmacol Ther 1997, 11 (suppl 3): 109-115. Such a controlled lag time could be obtained for instance by a programmed disintegration of the formulation due to erosion, dissolution or in general by components present in the formulation interacting with the environment in the gastro-intestinal tract. Preferably, the drug release from the dosage form could be triggered by the pH variation between jejunum and ileum.

Alternatively, the drug release from the dosage form can be chronographic controlled to obtain the above specified time limits, such as for instance described in the European Patent Application, Publication No. EP-A-0384642.

According to another aspect of the invention, a sustained release formulation can be constructed by any known principle, such as eroding or non-eroding matrices, membrane-coating layers or by diffusion or osmotically driven drug release. Suitable techniques for the construction of such formulations are for instance described in M. E. Aulton, Pharmaceutics, The science of dosage form design. (1988).

In the present invention an IBAT inhibitor compound is combined with a bile acid binder thereby avoiding a possible risk of excess of bile acids in colon caused by the inhibition of the ileal bile acid transport system. An excess of bile acids in the visceral contents may cause diarrhoea. Thus, the combination disclosed herein is also useful in the treatment of a possible side effect such as diarrhoea in patients during therapy comprising IBAT inhibitor compounds.

Suitable bile acid binders for such a combination therapy are resins, such as cholestyrmine, cholestipol or colesevelam. One advantage is that the dose of bile acid binder might be kept lower than the therapeutical dose for treatment of cholesterolemia in single treatment comprising solely a bile acid binder. By a low dose of bile acid binder any possible side effects caused by poor tolerance of the patient to the therapeutic dose could also be avoided.

The bile acid binder is administered in a dosage form with colon release, i.e. delivery of the active dose of bile acid binder in the colon. A possible risk of receiving an excess of bile acid in the colon by treatment with an IBAT inhibitor could be avoided by coadministration of a bile acid binder with colon release. Thus, any excess of bile acid in the colon, with a possible risk to cause diarrhoea, will be bound into a resin. The dose of the bile acid binder could be kept low due to an effective use of the dose by such a colon release. The colon delivery of the bile acid binder can be obtained by a formulation comprising a core containing the bile acid binder and optionally pharmaceutically acceptable excipients, and a coating of said core with a release membrane adapted for colonic delivery. Technologies to obtain such a delivery of drugs to the colon are for example described in Drug development and Industrial Pharmacy 1997, 23: 893-913.

Disclosed herein is a composition comprising one or more IBAT inhibitors of the invention and cholestyramin and/or colesevelam and/or cholestipol.

Disclosed herein is a composition comprising one or more of the compounds of Example 1, 2, 3, 4, 5, 6, 7, 8, 9, 10, 11, 12, 13 and 14 and cholestyramin and/or colesevelam and/or cholestipol.

Also disclosed herein is a composition comprising one or more of the compounds of 1,1-Dioxo-3,3-dibutyl-5-phenyl-7-methylthio-8-(N-{(R)-1'-phenyl-1'-[N'-(carboxymethyl)-carbamoyl]methyl}carbamoylmethoxy)-2,3,4,5-tetrahydro-1,5-benzothiazepine (Example 5) and cholestyramin and/or colesevelam and/or cholestipol.

Also disclosed herein is a composition comprising one or more of the compounds of 1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine (Example 13), and cholestyramin and/or colesevelam and/or cholestipol.

Also disclosed herein is a composition comprising one or more of the compounds of 1,1-Dioxo-3,3-dibutyl-5-phenyl-7-methylthio-8-(N-{ (R)-1'-phenyl-1'-[N'-(carboxymethyl) carbamoyl] methyl} carbamoylmethoxy)-2,3,4,5-tetrahydro-1,5-benzothiazepine (Example 14) and cholestyramin and/or colesevelam and/or cholestipol.

### Preparation of core material

The core material for the units, i.e. the tablets or the individual pellets can be constituted according to different principles. The core material may be homogenous or heterogeneous. The core containing the active principle may be differently formulated such as monolithic tablets, capsules, granules, pellets, other particles or crystals.

With a homogenous core material is meant, that it has a homogenous distribution of active substance throughout the core material.

Depending on if the IBAT inhibitor and the bile acid binder are formulated separately or combined, the active substances are optionally mixed with further components to obtain preferred handling and processing properties and a suitable concentration of the active substance in the final mixture. Such components can be binders, surfactants, lubricants, glidants, fillers, additives or other pharmaceutically acceptable ingredients, alone or in mixtures.

Said core material may be produced either by direct compression of the mixed ingredients, or by granulation of the ingredients followed by compression of the granulated material.

In direct compression, the ingredients are mixed and compressed by using ordinary tabletting equipment.

For the granulation there are numerous alternatives of granulating procedures mentioned in the literature, dry methods like roller compaction (Chilsonator) and wet methods utilizing granulating solutions with and without the addition of binders. A variant of the wet methods is to make a spray-granulation in a fluid bed.

For the wet granulating methods, either organic solvents, aqueous solutions or pure water may be utilized to prepare the granulating solutions. Due to environmental considerations pure water is preferred, if it is possible due to the composition of the mixture.

Homogenous core particles can also be prepared by techniques such as dry or wet milling, freeze milling, air-jet micronisation, spray drying, spray chilling, controlled crystallisation, supercritical crystallisation, emulsion solvent evaporation and emulsion solvent extraction.

The core material may also be produced by extrusion/spheronization, balling or compression, utilizing different process equipments.

The size of the formulated core materials is approximately between 2 and 20 mm, preferably between 3 and 15 mm for a tablet preparation, and between 0.001 and 4 mm, preferably between 0.001 and 2 mm for a pellet preparation.

The manufactured core material may be further layered with additional ingredients comprising the active substance and/or be used for further processing.

Alternatively, the core material may be heterogeneous with an inner zone, for instance a seed or sphere, not containing the active substance. A layer comprising the active substance, and optionally pharmaceutically acceptable excipients, surrounds this seed or sphere.

The seed or sphere may be soluble or insoluble. Optionally, the seed or sphere (inner zone) may be coated with an inert layer to prepare a smooth surface before the layer containing active substance is applied onto the seed/sphere.

Insoluble seeds/spheres may comprise different oxides, celluloses, organic polymers and other materials, alone or in mixtures. Water-soluble seeds/spheres may comprise different inorganic salts, sugars and other materials, alone or in mixtures. The size of the seeds may vary between approximately 0.1 and 2 mm. The seeds layered with the matrix containing the active substance are produced either by powder or solution/suspension layering using for instance granulating or spray coating/layering equipment.

### Processes for application of release modifying membranes

A release modifying membrane can be applied to the core material, being a monolithic tablet, multiple units or a hard or soft gelatine capsule, by coating or layering procedures in suitable equipment such as coating pans, coating granulators or in a fluidized bed apparatus using water and/or organic solvents for the coating process. Also powder-coating principles may be applied. Another possibility is to apply the coating by microencapsulation techniques such as coacervation, emulisification with subsequent removal of the solvent by extraction or evaporation, ionotropic gelation or congealing.

Such modifying release membranes may be applied on core material comprising the IBAT inhibitor for delivery to the distal small intestine and optionally also be applied to the bile acid binder for delivery to the colon.

### Pharmaceutical additives

Modifying release coatings may be obtained by one or more, separately or in compatible combinations of pharmaceutically acceptable ingredients, in amounts carefully titrated to reach the intended release properties. As delayed release coating layer, the following pH sensitive polymers can be applied; e.g. methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethyl ethylcellulose, shellac or other suitable enteric coating layer polymer(s). The coating layer may also be composed of film-forming polymers being sensitive to other luminal components than pH, such as bacterial degradation or a component that has such a sensitivity when it is mixed with another film-forming polymer. Examples of such components providing delayed release to the intended regions are; polymers comprising azo bond(s), polysaccharides such as pectin and its salts, galactomannans, amylose and chondroitin, disulphide polymers and glycosides.

The delayed release coating or an additional coating of the formulation may contain other film-forming polymers being non-sensitive to the luminal conditions for technical reasons or chronographic control of the drug release. Materials to be used for such purpose include sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures.

Additives such as dispersants, colorants, pigments, additional polymers e.g. poly(ethylacrylat, methylmethacrylat), anti-tacking and anti-foaming agents may also be included into the coating layer. Other compounds may be added to increase film thickness and to decrease diffusion of acidic gastric juices into the core material.

The coating layers may also contain pharmaceutically acceptable plasticizers to obtain desired mechanical properties. Such plasticizers are for instance, but not restricted to, triacetin, citric acid esters, phthalic acid esters, dibutyl sebacate, cetyl alcohol, polyethylene glycols, glycerol monoesters, polysorbates or other plasticizers and mixtures thereof. The amount of plasticizer is preferably optimized for each formula, in relation to the selected polymer(s), selected plasticizer(s) and the applied amount of said polymer(s).

In preparation of tablets, either as monolithic drug containing cores for subsequent coating with a modified release membrane or as a matrix for coated multiple units, additional ingredients may be needed to obtain suitable technical properties such as binders, disintegrants, bulk agents, glidants, lubricants, and coatings agents without effects on the drug release such as water soluble polymers, anti-tacking agents, colourants, pigments and waxes. Ingredients well known for such usage are for example described in "Handbook of pharmaceutical excipients", 2nd edition, 1994, Pharmaceutical Press, London.

### Preparation of final dosage forms

Coated units may be filled into hard gelatine capsules or mixed with tablet excipients, such as fillers, binders, disintegrants, lubricants and other pharmaceutically acceptable additives, and be compressed into tablets. The compressed tablet is optionally covered with film-forming agents to obtain a smooth surface of the tablet and further enhance the mechanical stability of the tablet during packaging and transport. Such a tablet coat, which may be applied on a multiple unit tablet or a conventional tablet, may further comprise additives like anti-tacking agents, colourants and pigments or other additives to improve the tablet appearance.

Suitable drugs for the new formulations are IBAT inhibitor compounds of formula II such as described in the above-discussed documents.

A combination according to the invention should preferably comprise simultaneous, separate or sequential administration of an IBAT inhibitor compound of formula II and a bile acid binder. The IBAT inhibitor could preferably be formulated for ileum delivery and the bile acid binder could preferably be formulation for colon release.

### Dosage

A suitable unit dose will vary with respect to the patient's body weight, condition and disease severity. The dose will also depend on if it is to be used for prophylaxis or in the treatment of severe conditions, as well as the route of administration. The daily dose can be administered as a single dose or divided into two or more unit doses. An orally administered daily dose of an IBAT inhibitor is preferably within 0.1 - 5,000 mg, e.g. 0.01-1000 mg, such as 0.1-800 mg more preferable 1 - 500 mg e.g. 100 - 400 mg.

A pharmaceutical formulation according to the present invention with a targeted delivery in the gastro intestinal tract provides a reduced systemic exposure, as can be measured by the area under the drug plasma concentration versus time curve (AUC), while maintaining or even increasing the therapeutic effect, as e.g. measured by serum cholesterol reduction.

A combination therapy comprising an IBAT inhibitor of formula II and a bile acid binder comprises preferably a low daily dose of the bile acid binder, such as less than 5 g of a resin, and more preferably less than 4, 3, 2 or less than 1 g. Suitable ranges may be 0,1-5 g, 0.5- 4 g, 1-3 g, 2-4 g, 2-3 g per day. A dosage form with colon release of the bile acid binder could be constructed by any of the above described principles for delayed release formulations.

A tablet may consist of an inner core of 1-1000 mg, e.g. 200-800 mg, 50-400 mg, 10-200 mg or 20-80 mg acid binder in a colonic delivery formulation and an outer lamina with 1-100 mg, 5-50 mg e.g. 1-20 mg of an IBAT inhibitor.

The daily dose of IBAT inhibitor and /or bile acid binder can be administered as a single dose or divided into one, two, three or more unit doses.

Dosing three times a day with 400 mg of colesevelam in a colonic release formulation will give an adequate binding of bile acids in the colon as the total luminal volume is expected to be about 100 ml, which is in accordance to an accepted pharmacokinetic calculation volume of 250 to 300 ml for the small gut. The daily recommended total dose of colesevelam to block bile acid absorption in total gut of humans is 3750 mg/day.

The predicted dose to treat constipation with Example 14 is 10 mg/day, as full efficacy in lowering of bile acids in the human body is targeted a total dose of 20 mg/day is predicted to be adequate, which will induce colonic side effects in accordance with table 2. The tablet will consist of an inner core of 400 mg colesevelam in a colonic delivery formulation and an outer lamina with 7 mg of Example 14 or 0.7 mg of Examples 5, 8 or 13 in an immediate release form.

Disclosed herein is a kit comprising a compound of formula (I), or a pharmaceutically acceptable salt thereof, and a bile acid binder.

Also disclosed herein is a kit comprising a an IBAT inhibitor compound, or a pharmaceutically acceptable salt thereof, and a bile acid binder in the same or separate pharmaceutical formulation and possibly also an instruction for use.

The following contemplated Examples are intended to illustrate the invention.

The expression "comprising" as used herein should be understood to include, but not be limited to, the stated items.

### Example 1

1,1-Dioxo-3,3-dibutyl-5-phenyl-7-methylthio-8-(N-{(R)-α-[N-(carboxymethyl)carbamoyl] benzyl}carbamoylmethoxy)-2,3,4,5-tetrahydro-1,2,5-benzothiadiazepine, Mw. 696,89. This compound is prepared as described in Example 2 of WO3022286.

### Example 2

1,1-Dioxo-3,3-dibutyl-5-phenyl-7-methylthio-8- (N-{(R)-□α-[N'-((S)-1-carboxyethyl)-carbamoyl]benzyl}carbamoylmethoxy)-2,3,4,5-tetrahydro-1,5-benzothiazepine, Mw. 709,92.
This compound is prepared as described in Example 2 of WO03106482.

### Example 3

1,1-Dioxo-3,3-dibutyl-5-phenyl-7-methylthio-8-(*N*-{(R)-α-[*N*-((S)-1-carboxypropyl)-carbamoyl]benzyl}carbamoylmethoxy)-2,3,4,5-tetrahydro-1,2,5-benzothiadiazepine, Mw. 724,94.
This compound is prepared as described in Example 6 of WO3022286.

### Example 4

1,1-Dioxo-3,3-dibutyl-5-phenyl-7-methylthio-8-(*N*-{(R)-α-[*N*-((R)-1-carboxy-2-methylthioethyl)carbamoyl]benzyl}carbamoylmethoxy)-2,3,4,5-tetrahydro-1,2,5-benzothiadiazepine, Mw. 757,01.
This compound is prepared as described in Example 7 of WO3022286.

### Example 5

1,1-Dioxo-3,3-dibutyl-5-phenyl-7-methylthio-8-(*N*-{(R)-α-[*N*-((S)-1-carboxypropyl)-carbamoyl]-4-hydroxybenzyl}carbamoylmethoxy)-2,3,4,5-tetrahydro-1,2,5-benzothiadiazepine, Mw. 740,94.
This compound is prepared as described in Example 29 of WO3022286.

### Example 6

1,1-Dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine, Mw. 773,00.
This compound is prepared as described in Example 30 of WO3022286.

### Example 7

1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine, Mw. 738,97.
This compound is prepared as described in Example 15 of WO3022286.

### Example 8

1,1-Dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine, Mw. 756,94.
This compound is prepared as described in Example 26 of WO3022286.

### Example 9

1,1-Dioxo-3,3-dibutyl-5-phenyl-7-methylthio-8-(*N*-{(R)-α-[*N*-((S)-1-carboxybutyl)-carbamoyl]-4-hydroxybenzyl}carbamoylmethoxy)-2,3,4,5-tetrahydro-1,2,5-benzothiadiazepine, Mw. 754,97.
This compound is prepared as described in Example 28 of WO3022286.

### Example 10

1,1-Dioxo-3,3-dibutyl-5-phenyl-7-methylthio-8-(*N*-{(R)-α-[*N*-((S)-1-carboxyethyl)-carbamoyl]benzyl}carbamoylmethoxy)-2,3,4,5-tetrahydro-1,2,5-benzothiadiazepine, Mw. 710,91.
This compound is prepared as described in Example 5 of WO3022286.

### Example 11

1,1-Dioxo-3,3-dibutyl-5-phenyl-7-methylthio-8-(N-{(R)-□α-[N'-((S)-1-carboxypropyl)-carbamoyl]-4-hydroxybenzyl} carbamoylmethoxy)-2,3,4,5-tetrahydro-1,5-benzothiazepine, Mw. 739,95.
This compound is prepared as described in Example 1 of WO3022286.

### Example 12

1,1-Dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine, Mw. 726,91.
This compound is prepared as described in Example 11 of WO3022286.

### Example 13

1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine, Mw. 754,97.
This compound is prepared as described in Example 27 of WO3022286.

### Example 14

1,1-Dioxo-3,3-dibutyl-5-phenyl-7-methylthio-8-(N-{(R)-1'-phenyl-1'-[N'-(carboxymethyl)-carbamoyl]methyl}carbamoylmethoxy)-2,3,4,5-tetrahydro-1,5-benzothiazepine, Mw. 695,90.
This compound is prepared as described in Example 43 of WO0250051.

### Example 15: Pharmaceutical effect Mean Inhibitor effect (%)

ISBT Hu HEK Uptake SPA 13203 IBAT HUM Ileal Bile Acid Transporter Human HEK Glycocholic acid Uptake Radiometric - SPA Inhibitor IC50 Mean IC50 (nM) was determined for the compounds of examples 1-14

### Test system

### Animals

Species Mouse; Strain ApoE knock out; Sub strain C57BL/6; Sex Female; Total No. of animals 70; Body weight range 20 g to 22 g; Supplier Möllegaard's Breeding (Skensved, Denmark); Identification method ID cards (bar code).

Acclimatisation: At least one week at the Section of Laboratory; Animal Resource at AstraZeneca; Housing conditions: Kept five by five in cages (Makrolon III, 7 dm2) in a room with regulated temperature (22°C), relative humidity (40% to 60%) and a 12/12 hours light/dark cycle. Diet: Free access to R3 pellets (Lactamin, Vadstena, Sweden) during the housing and experimental period. Water: Free access to tap water during the housing and experimental period.

Bedding: Sprinkle bedding of aspen wood (Tapvei, Finland).

### Experimental procedures

The animals were orally administered vehicle (n=3)or the compound of Example 14 (0.156 (n=3), 0.625 (n=3) or 2.5 µmol/kg (n=3)) at 13:00 o'clock on the experimental day. Thirty minutes later, a trace amount of ⁷⁵SeHCAT (⁷⁵Se-homo-tauro-cholic acid) (0.1 mCi per 0.1 mL per mouse) was orally given to each mouse. Twenty-four hours after ⁷⁵SeHCAT administration, the animals were killed by CO2 inhalation. At sacrifice, the gall bladder and the whole intestine were removed, and the faeces during the 24-hour period after ⁷⁵SeHCAT administration was collected for each mouse. The gamma radioactivities of ⁷⁵SeHCAT in the faeces and in the gall bladder-intestine were separately counted by 1282 CompuGamma CS Gamma counter (Wallac oy, Turku, Finland). The stability as well as the quantity of the ⁷⁵SeHCAT administered to each mouse, were controlled with an additional ⁷⁵SeHCAT aliquot following the same experimental process as other tested samples in the study.

### Data analysis

The sum of the gamma counts from both the faeces and the gall bladder-intestine was considered as the total recovered ⁷⁵SeHCAT, which was averaged around 85% of the total ⁷⁵SeHCAT administered to each mouse. Of the recovered radioactivity of ⁷⁵SeHCAT, the percentage of the ⁷⁵SeHCAT detected in the faeces was considered as the faecal excretion while that in the gall bladder-intestine as body retention. Inhibitory effect of the compound of Example 14 on ⁷⁵SeHCAT intestinal absorption was calculated following the ⁷⁵SeHCAT body retention and the faecal excretion, and the ED50 of the compound was estimated following the dose-effect curve.

### Results

The mean IBAT inhibitory effect (%) at a dose (µmol/kg): 0.156 was determined for the compounds of examples 1-14 and is reported in Table 1

**Table 1**

| **Example** | **Structure** | **% inhibition 0.156 µmol/kg** | **Mean IC50 nM** |
|---|---|---|---|
| 1. | 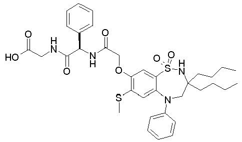 | 43 | 0,45 |
| 2. | 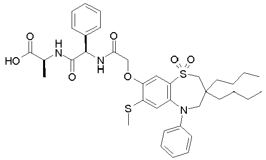 | 55 | 0,39 |
| 3. | 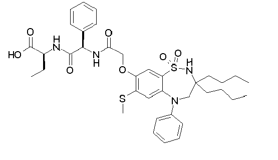 | 63 | 0,18 |
| 4. | 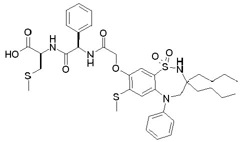 | 63 | 0,35 |
| 5. | 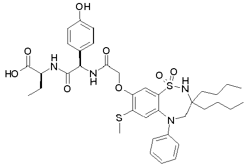 | 74 | 0,16 |
| 6. | 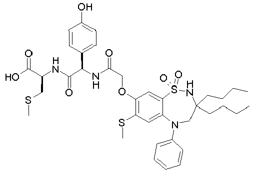 | 59 | - |
| 7. | 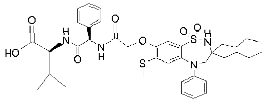 | 66 | 0,36 |
| 8. | 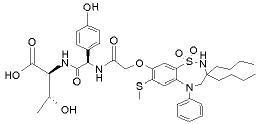 | 46 | 0,11 |
| 9. | 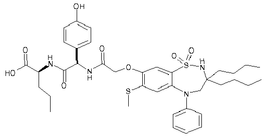 | 67 | - |
| 10. | 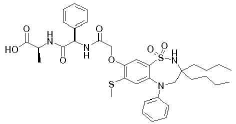 | 68 | 0,2 |
| 11. | 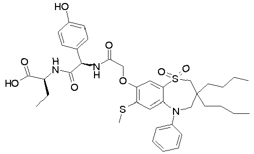 | 63 | 0,15 |
| 12. | 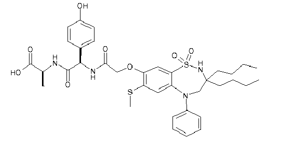 | 63 | 0,3 |
| 13. | 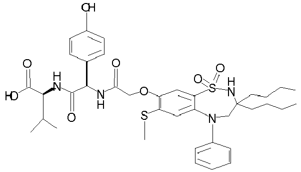 | 68 | 0,13 |
| 14. | 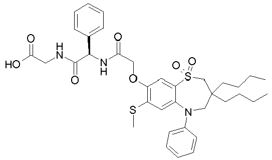 | 28 | 1,2 |

### Example 16

A Phase IIb, Double-blind, Randomized, Placebo-controlled, Multi-centre, Dose-finding, Efficacy and Safety Study of a Range of Doses of the compound of Example 14 in about 200 patients with Chronic Idiopathic Constipation, where patients were treated for 56 days, was performed. The patients were males or non-pregnant females ≥20 years of age and ≤80 years of age, having a body mass index (BMI) ≥18.5 but <35.

All patients with adverse events of diarrhea and abdominal pain type related to treatment with the compound of Example 14 are presented in table 2.

| **Table 2 Number patients with diarrhea and abdominal pain in a treatment study of Chronic idiopathic Constipation** | | | | | |
|---|---|---|---|---|---|
| **Dose** | | **Placebo** | **5 mg** | **10 mg** | **15 mg** |
| Diarrhoea | Number of Patients,(% of Population) | 2 (4.3) | 4 (8.3) | 5 (10.6) | 8 (16.7) |
| Abdominal Pain | Number of Patients,(% of Population) | 0 (0.0) | 5 (10.4) | 5 (10.6) | 13 (27.1) |

Contradictory patients receiving also colonic delivery of 400mg 3 times a day of "colesevelam" showed little increase in the two bile acid dependent side effects generated from colon.

Thus, the compound of Example 14 in combination with the bile acid binder "colesevelam" turned out to effectively block bile acid side effects generated from the colon.

### Example 17

A formulation for delayed release of the IBAT inhibitor having the following composition is prepared:

| Substance | amount/capsule (mg) |
|---|---|
| | |
| IBAT inhibitor compound | |
| Example 14 | 10 |
| | |
| Non pareil spheres | 500 |
| Ethyl cellulose | 2 |
| Hydroxypropylmethyl cellulose | 10 |
| Eudragit L100-55 (CAS No. 25212-88-8) | 25 |
| Triethylcitrate | 2.4 |

The active drug is dissolved together with ethyl cellulose and hydroxypropyl cellulose in ethanol 99 %.

The mixture is then sprayed onto the non-pareil spheres in a fluidized bed apparatus. Thereafter, the pellets are dried and aerated to remove residual ethanol. The Eudragit L100-55 dispersion with addition of triethyl citrate is then sprayed onto the drug beads in a fluidized bed apparatus. Subsequently, the coated beads are filled into hard gelatine capsules after drying and sieving.

### Example 18

A formulation for delayed release of the IBAT inhibitor having the following composition is prepared:

| | |
|---|---|
| Ingredient | amount/tablet (mg) |
| IBAT inhibitor compound | |
| Example 14 | 10 |
| | |
| Silicon dioxide | 200 |
| Povidone K-25 | 20 |
| Eudragit FS30D (CAS No 26936 - 24 - 3) | 30 |
| Microcrystalline cellulose | 250 |
| Sodium stearyl fumarate | 5 |

The active drug is suspended in water and sprayed onto silicon dioxide cores of a predefined size in a fluidized bed apparatus. The drug pellets are dried in an oven at 40° C for 24 h. Thereafter, a layer of Povidone K-25 is applied onto the beads from an ethanolic solution in a fluidized bed apparatus. A final coat of Eudragit FS30D dispersion is thereafter applied in a fluidized bed. The coated beads are mixed with microcrystalline cellulose and sodium stearyl fumarate in a mixer and subsequently compressed to tablets.

### Example 19

A combination tablet comprising an IBAT inhibitor of Formula (I) or Formula (II) as described above, and colesevelam, having an immediate release of the IBAT inhibitor and colon release of the bile acid binder, is prepared.

**Ingredient amount/tablet (mg)**

| *Core* | |
|---|---|
| Colesevelam hydrochloride | 400 |
| Microcrystalline cellulose | 150 |
| Hydroxypropyl methyl cellulose | 50 |
| Colloidal silicon dioxide | 10 |
| Magnesium stearate | 5 |
| *Colon release layer* | |
| Eudragit FS30D | 60 |
| PlasACRYL T20 (CAS No 123-94-4) | 6 |
| *IBAT inhibitor layer* | |
| IBAT inhibitor of Example 14 | 7 |
| Hydroxypropylmethyl cellulose | 12 |
| Croscarmellose sodium | 6 |
| *Protective coating* | |
| Hydroxypropylmethyl cellulose | 12 |
| Polyethylene glycol | 2 |

Colesevelam hydrochloride, microcrystalline cellulose and colloidal silicon dioxide are mixed and granulated with hydroxypropyl methyl cellulose dissolved in water. The granules are dried and mixed with magnesium stearate and compressed into tablets. The EUDRAGIT FS30D dispersion and water are stirred into the PlasACRYL T20 and sprayed onto the core tablets using a suitable coating machine. The IBAT inhibitor coating suspension is prepared by mixing the IBAT inhibitor, hydroxypropyl methyl cellulose and croscarmellose sodium in water and sprayed onto the tablet cores with the colon release layer using a suitable coating machine. Finally the protective coating solution of hydroxypropylmethyl cellulose and polyethylene glycol is sprayed onto the tablets using a suitable coating machine.

### Example 20

A Colesevelam colon release tablet having the following composition is prepared:

| Ingredient amount /tablet (mg) | |
|---|---|
| Core | |
| Colesevelam hydrochloride | 400 |
| Microcrystalline cellulose | 150 |
| Hydroxypropyl methyl cellulose | 50 |
| Colloidal silicon dioxide | 10 |
| Magnesium stearate | 5 |
| Colon release layer | |
| Amylose30 | |
| Eudragit S100 | 60 |
| Triethylcitrate | 6 |
| Glycerolmonostearate | 3 |

Colesevelam hydrochloride, microcrystalline cellulose and colloidal silicon dioxide are mixed and granulated with hydroxypropyl methyl cellulose dissolved in water. The granules are dried and mixed with magnesium stearate and compressed to tablets. Amylose, Eudragit 100, triethylcitrate and glycerolmonosterate are dissolved in suitable solvents and sprayed onto the tablet cores using a suitable coating machine.

### Example 21

This study is performed in 4 male beagle dogs (weight approximately10 kg). A commercially available diet, Teklad 2021 from Harlan Teklad-Europe, Blackthorn, England, is offered in portions of 175 g per animal twice daily with an equal amount of water added.

The animals are trained in the procedures using a commercially available diet, GLP-Diet from Dechra Veterinary Products, Denmark, as a reward. The amount used per animal is minimal and is not recorded.

On dosing days (Days 1 and 4) the animals are offered 50 g GLP diet during the rectal dosing procedure and the remaining meal (125 g) was given 1 hour after the rectal dosing procedure.

Example 5 substance (30 mg /kg, 5 mL/kg suspension in 20% v/v propylene glycol in purified water) is administered by oral dosing according to the most recent body weight data. All groups receive Example 5 substance orally in combination with rectal administration of cholestyramine or placebo.

Rectal administration of cholestyramin (total 1.2-2.2 g, 12 mL suspension in water) and a placebo formulation (exploration gel) are used in order to comply with the intended human route of administration. Cholestyramin and placebo is given by rectal catheterisation using a 40 cm flexible plastic tube (unomedical "feeding tube") in order to apply the test substances into the proximal part of colon. Treatment is performed on Day 1 and Day 4

Following each dosing occasion, all animals are continuously monitored for a period of 6 hours. The number of defaecations is counted. Faeces consistency is evaluated using the Bristol Stool form Scale (seven grades scale; lowest 1, separate hard lumps, like nuts (hard to pass); highest 7, watery, no solid pieces, entirety liquid). The amount of passed faeces for each defecation event is scored according to the following grading system: 1-minimal, 2-slight, 3-moderate, 4-marked.

Score data is analysed with the t-test. Significant inter-group differences are detected, the subsequent identification of the groups is carried out with one-sided t-test.-For all tests, the level of significance is defined as p<0.05.

The placebo and cholestyramin groups provide a Bristol Stool form Scale (BSS) mean value of 6.7+-0.3, 5.3+-0.6, (mean+-SEM), respectively. This difference is statistical significant on the significance level p≤0.05. The placebo group has watery faeces, as expected the cholestyramin group has a more solid faeces.

The placebo and cholestyramin group pass the following amount of faeces according to the scale above for each defaecation event and provide a mean value of 3.5+-0.6, 2.3+-0.8, (mean+-SEM), respectively. This difference is not statistical significant on the significance level p≤0.05. The placebo treated group pass more amount of faeces compared with the cholestyramin group, even if not significant. Both parameters BSS and "pass amount of faeces" indicates that the colestyramin group has lower free bile acid concentrations within in colon compared with the placebo group. As bile acids in the colon have a laxative effect this experiment confirms that a colon release of bile acid binders works according to the invention.

## Claims

1. A combination comprising an IBAT inhibitor of formula II 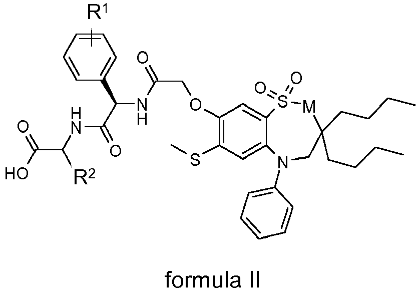 wherein
M is -CH₂ or NH;
**R¹** is H or OH; and
**R²** is H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, CH₂OCH₃, -CH(OH)CH₃, -CH₂SCH₃, or -CH₂CH₂-S-CH₃; or a pharmaceutically acceptable salt thereof,
and a bile acid binder,
for simultaneous, sequential or separate administration, wherein the bile acid binder is formulated for colon release, for use in the treatment of a liver disease selected from the group consisting of primary biliary cirrhosis (PBC); progressive familial intrahepatic cholestasis (PFIC); Alagilles syndrome (ALGS); primary sclerosing cholangitis (PSC); non-alcoholic steatohepatitis (NASH); and pruritus of cholestatic liver disease.

2. A combination for use according to claim 1, wherein the liver disease is primary biliary cirrhosis (PBC).

3. A combination for use according to claim 1, wherein the liver disease is progressive familial intrahepatic cholestasis (PFIC).

4. A combination for use according to claim 1, wherein the liver disease is Alagilles syndrome (ALGS).

5. A combination for use according to claim 1, wherein the liver disease is primary sclerosing cholangitis (PSC).

6. A combination for use according to claim 1, wherein the liver disease is non-alcoholic steatohepatitis (NASH).

7. A combination for use according to claim 1, wherein the liver disease is pruritus of cholestatic liver disease.

8. A combination for use according to any one of claims 1 to 7, wherein the combination is designed to deliver the IBAT inhibitor in the small intestine and the bile acid binder in the colon.

9. A combination for use according to any one of claims 1 to 8, wherein the compound of formula (II) is selected from the group consisting of:
1,1-Dioxo-3,3-dibutyl-5-phenyl-7-methylthio-8-(N-{(R)-α-[N-(carboxymethyl)carbamoyl] benzyl} carbamoylmethoxy)-2,3,4,5-tetrahydro-1,2,5-benzothiadiazepine;
1,1-Dioxo-3, 3-dibutyl-5-phenyl-7-methylthio-8-(N-{(R)-α-[N'-((S)-1-carboxyethyl) carbamoyl] benzyl} carbamoylmethoxy)-2, 3,4, 5-tetrahydro-1, 5-benzothiazepine;
1,1-Dioxo-3,3-dibutyl-5-phenyl-7-methylthio-8-(*N*-{(R)-α-[*N*-((S)-1-carboxypropyl) carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthioethyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxybutyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxyethyl) carbamoyl]benzyl}carbamoylmethoxy)-2,3,4,5-tetrahydro-1,2,5-benzothiadiazepine;
1,1-dioxo-3, 3-dibutyl-5-phenyl-7-methylthio-8- (N-{(R)-α-[N'-((S)-1-carboxypropyl) carbamoyl]-4-hydroxybenzyl} carbamoylmethoxy) -2, 3,4, 5-tetrahydro-1, 5-benzothiazepine;
1,1-Dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine; and
1,1-Dioxo-3, 3-dibutyl-5-phenyl-7 -methylthio-8-(N-{(R)-1'-phenyl-1'-[N'-(carboxymethyl) carbamoyl]methyl}carbamoylmethoxy)-2,3,4,5-tetrahydro-1,5-benzothiazepine,
or a pharmaceutically acceptable salt thereof.

10. A combination for use according to claim 9, wherein the compound of formula (II) is 1,1-dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine, or a pharmaceutically acceptable salt thereof.

11. A combination for use according to any one of claims 1 to 10, wherein the bile acid binder is cholestyramine, cholestipol or colesevelam.

12. A pharmaceutical formulation comprising an IBAT inhibitor of formula II 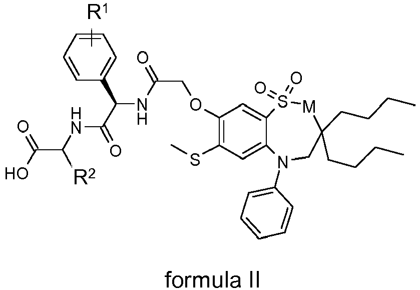 wherein
**M** is -CH₂ or NH;
**R¹** is H or OH; and
**R²** is H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, CH₂OCH₃, -CH(OH)CH₃, -CH₂SCH₃, or -CH₂CH₂-S-CH₃; or a pharmaceutically acceptable salt thereof,
and a bile acid binder,
wherein the bile acid binder is in the core and formulated for release in the colon and wherein the IBAT inhibitor is in an outer layer formulated for immediate release or for delayed release in the distal jejunum or the proximal ileum, for use in the treatment of a liver disease selected from the group consisting of primary biliary cirrhosis (PBC); progressive familial intrahepatic cholestasis (PFIC); Alagilles syndrome (ALGS); primary sclerosing cholangitis (PSC); non-alcoholic steatohepatitis (NASH); and pruritus of cholestatic liver disease.

13. A formulation for use according to claim 12, comprising
(i) an inner core comprising a bile acid binder;
(ii) a colon release layer onto the core;
(iii) an IBAT inhibitor layer onto said colon release layer; and
(iv) an outer protective coating.

14. A formulation for use according to claim 12 or 13, wherein the IBAT inhibitor layer comprises a compound selected from the group consisting of:
1,1-Dioxo-3,3-dibutyl-5-phenyl-7-methylthio-8-(N-{(R)-α-[N-(carboxymethyl)carbamoyl] benzyl} carbamoylmethoxy)-2,3,4,5-tetrahydro-1,2,5-benzothiadiazepine;
1,1-Dioxo-3,3-dibutyl-5-phenyl-7-methylthio-8-(N-{(R)-α-[N'-((S)-1-carboxyethyl) carbamoyl] benzyl} carbamoylmethoxy)-2,3,4,5-tetrahydro-1,5-benzothiazepine;
1,1-Dioxo-3,3-dibutyl-5-phenyl-7-methylthio-8-(*N*-{(R)-α-[*N*-((S)-1-carboxypropyl) carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthioethyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxybutyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxyethyl) carbamoyl]benzyl}carbamoylmethoxy)-2,3,4,5-tetrahydro-1,2,5-benzothiadiazepine;
1,1-dioxo-3,3-dibutyl-5-phenyl-7-methylthio-8-(N-{(R)-α-[N'-((S)-1-carboxypropyl) carbamoyl]-4-hydroxybenzyl}carbamoylmethoxy)-2,3,4,5-tetrahydro-1,5-benzothiazepine;
1,1-Dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxy-2-methylpropyl)carbamoyl]-4-hydroxybenzyl}carbamoylmethoxy)-2,3,4,5-tetrahydro-1,2,5-benzothiadiazepine; and
1,1-Dioxo-3,3-dibutyl-5-phenyl-7-methylthio-8-(N-{(R)-1'-phenyl-1'-[N'-(carboxymethyl) carbamoyl]methyl}carbamoylmethoxy)-2,3,4,5-tetrahydro-1,5-benzothiazepine,
or a pharmaceutically acceptable salt thereof.

15. A formulation for use according to claim 14, wherein the IBAT inhibitor layer comprises the compound 1,1-dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine, or a pharmaceutically acceptable salt thereof.

16. A formulation for use according to any one of claims 12 to 15, wherein the bile acid binder is cholestyramine, cholestipol or colesevelam.

## Patentansprüche

1. Kombination, umfassend einen IBAT-Hemmer der Formel II, 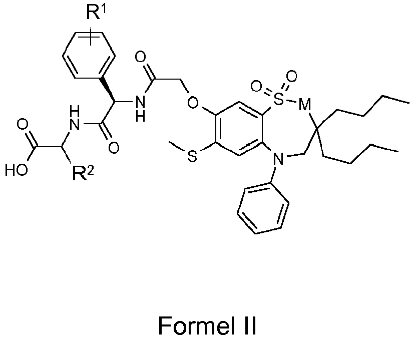 wobei es sich bei
**M** um CH₂ oder NH;
**R¹** um H oder OH; und
**R²** um H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, -CH₂OCH₃, -CH(OH)CH₃, -CH₂SCH₃ oder -CH₂CH₂-S-CH₃ handelt,
oder ein pharmazeutisch verträgliches Salz davon,
und einen Gallensäurebinder,
zur gleichzeitigen, sequenziellen oder separaten Anwendung, wobei der Gallensäurebinder zur Freisetzung im Colon formuliert ist, zur Verwendung bei der Behandlung einer Lebererkrankung, ausgewählt aus der Gruppe, die aus primär biliärer Zirrhose (PBC), progressiver familiärer intrahepatischer Cholestase (PFIC), Alagille-Syndrom (ALGS), primär sklerosierender Cholangitis (PSC), nichtalkoholischer Steatohepatitis (NASH) und Pruritus bei cholestatischer Lebererkrankung besteht.

2. Kombination zur Verwendung nach Anspruch 1, wobei es sich bei der Lebererkrankung um primär biliäre Zirrhose (PBC) handelt.

3. Kombination zur Verwendung nach Anspruch 1, wobei es sich bei der Lebererkrankung um progressive familiäre intrahepatische Cholestase (PFIC) handelt.

4. Kombination zur Verwendung nach Anspruch 1, wobei es sich bei der Lebererkrankung um das Alagille-Syndrom (ALGS) handelt.

5. Kombination zur Verwendung nach Anspruch 1, wobei es sich bei der Lebererkrankung um primär sklerosierende Cholangitis (PSC) handelt.

6. Kombination zur Verwendung nach Anspruch 1, wobei es sich bei der Lebererkrankung um nichtalkoholische Steatohepatitis (NASH) handelt.

7. Kombination zur Verwendung nach Anspruch 1, wobei es sich bei der Lebererkrankung um Pruritus bei cholestatischer Lebererkrankung handelt.

8. Kombination zur Verwendung nach einem der Ansprüche 1 bis 7, wobei die Kombination dafür ausgelegt ist, den IBAT-Hemmer im Dünndarm und den Gallensäurebinder im Colon abzugeben.

9. Kombination zur Verwendung nach einem der Ansprüche 1 bis 8, wobei die Verbindung der Formel (II) aus der Gruppe ausgewählt ist, die aus Folgendem besteht:
1,1-Dioxo-3,3-dibutyl-5-phenyl-7-methylthio-8-(*N*-{(*R*)-α-[*N-*(carboxymethyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(R)-α-[*N'*-((S)-1-carboxyethyl)carbamoyl]benzyl}carbamoylmethoxy)-2,3,4,5-tetrahydro-1,5-benzothiazepin;
1,1-Dioxo-3,3-dibutyl-5-phenyl-7-methylthio-8-(*N*-{(R)-α-[*N*-((S)-1-carboxypropyl)carbamoyl]benzyl}carbamoylmethoxy)-2,3,4,5-tetrahydro-1,2,5-benzoth iad iazepin;
1,1-Dioxo-3,3-dibutyl-5-phenyl-7-methylthio-8-(*N*-{(*R*)-α-[*N*-((*R*)-1-carboxy-2-methylthioethyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxypropyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*R*)-1-carboxy-2-methylthioethyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxy-2-(*R*)-hydroxypropyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxybutyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxyethyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N'*-((*S*)-1-carboxypropyl)carbamoyl]-4-hydroxybenzyl}carbamoylmethoxy)-2,3,4,5-tetrahydro-1,5-benzothiazepin;
1,1-Dioxo-3,3-dibutyl-5-phenyl-7-methylthio-8-(*N*-{(*R*)-α-[*N*-((*S*)-1-carboxyethyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxy-2-methylpropyl)carbamoyl]-4-hydroxybenzyl}carbamoylmethoxy)-2,3,4,5-tetrahydro-1,2,5-benzothiadiazepin und
1,1-Dioxo-3,3-dibutyl-5-phenyl-7-methylthio-8-(*N*-{(*R*)-1'-phenyl-1'-[*N*'-(carboxymethyl)carbamoyl]methyl}carbamoylmethoxy)-2,3,4,5-tetrahydro-1,5-benzoth iazepin
oder ein pharmazeutisch verträgliches Salz davon.

10. Kombination zur Verwendung nach Anspruch 9, wobei es sich bei der Verbindung der Formel (II) um 1,1-Dioxo-3,3-dibutyl-5-phenyl-7-methylthio-8-(*N-*{(*R*)-α-[*N*-((*S*)-1-carboxypropyl)carbamoyl]-4-hydroxybenzyl}carbamoylmethoxy)-2,3,4,5-tetrahydro-1,2,5-benzothiadiazepin oder ein pharmazeutisch verträgliches Salz davon handelt.

11. Kombination zur Verwendung nach einem der Ansprüche 1 bis 10, wobei es sich bei dem Gallensäurebinder um Colestyramin, Colestipol oder Colesevelam handelt.

12. Pharmazeutische Formulierung, umfassend einen IBAT-Hemmer der Formel II, 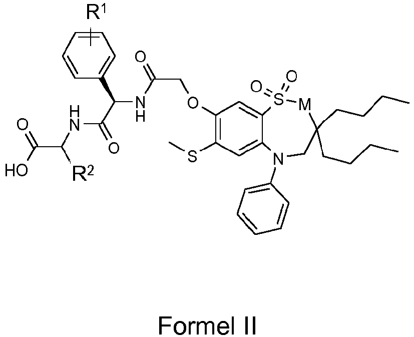 wobei es sich bei
**M** um CH₂ oder NH;
**R¹** um H oder OH; und
**R²** um H, -CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, -CH₂OCH₃, -CH(OH)CH₃, -CH₂SCH₃ oder -CH₂CH₂-S-CH₃ handelt,
oder ein pharmazeutisch verträgliches Salz davon,
und einen Gallensäurebinder,
wobei sich der Gallensäurebinder im Kern befindet und zur Freisetzung im Colon formuliert ist und wobei sich der IBAT-Hemmer in einer außenliegenden Schicht befindet und zur sofortigen Freisetzung oder zur verzögerten Freisetzung im distalen Jejunum oder dem proximalen Ileum formuliert ist, zur Verwendung bei der Behandlung einer Lebererkrankung, ausgewählt aus der Gruppe, die aus primär biliärer Zirrhose (PBC), progressiver familiärer intrahepatischer Cholestase (PFIC), Alagille-Syndrom (ALGS), primär sklerosierender Cholangitis (PSC), nichtalkoholischer Steatohepatitis (NASH) und Pruritus bei cholestatischer Lebererkrankung besteht.

13. Formulierung zur Verwendung nach Anspruch 12, welche Folgendes umfasst:
(i) einen innenliegenden Kern, der einen Gallensäurebinder umfasst;
(ii) eine Colon-Freisetzungsschicht auf dem Kern;
(iii) eine IBAT-Hemmer-Schicht auf der Colon-Freisetzungsschicht; und
(iv) eine außenliegende Schutzschicht.

14. Formulierung zur Verwendung nach Anspruch 12 oder 13, wobei die IBAT-Hemmer-Schicht eine Verbindung umfasst, die aus der Gruppe ausgewählt ist, welche aus Folgendem besteht:
1,1-Dioxo-3,3-dibutyl-5-phenyl-7-methylthio-8-(N-{(*R*)-α-[*N-*(carboxymethyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N'*-((*S*)-1-carboxyethyl)carbamoyl]benzyl}carbamoylmethoxy)-2,3,4,5-tetrahydro-1,5-benzothiazepin;
1,1-Dioxo-3,3-dibutyl-5-phenyl-7-methylthio-8-(*N*-{(*R*)-α-[*N*-((*S*)-1-carboxypropyl)carbamoyl]benzyl}carbamoylmethoxy)-2,3,4,5-tetrahydro-1,2,5-benzoth iad iazepin;
1,1-Dioxo-3,3-dibutyl-5-phenyl-7-methylthio-8-(*N*-{(*R*)-α-[*N*-((*R*)-1-carboxy-2-methylthioethyl)carbamoyl]benzyl}carbamoylmethoxy)-2,3,4,5-tetrahydro-1,2,5-benzoth iad iazepin;
1,1-Dioxo-3,3-dibutyl-5-phenyl-7-methylthio-8-(*N*-{(*R*)-α-[*N*-((*S*)-1-carboxypropyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*R*)-1-carboxy-2-methylthioethyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxy-2-methylpropyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxy-2-(*R*)-hydroxypropyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxybutyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxyethyl)carbamoyl]benzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N'*-((*S*)-1-carboxypropyl)carbamoyl]-4-hydroxybenzyl}carbamoylmethoxy)-2,3,4,5-tetrahydro-1,5-benzothiazepin;
1,1-Dioxo-3,3-dibutyl-5-phenyl-7-methylthio-8-(*N*-{(*R*)-α-[*N*-((*S*)-1-carboxyethyl)carbamoyl]-4-hydroxybenzyl}carbamoylmethoxy)-2,3,4,5-tetrahydro-1,2,5-benzothiadiazepin;
1,1-Dioxo-3,3-dibutyl-5-phenyl-7-methylthio-8-(*N*-{(*R*)-α-[*N*-((*S*)-1-carboxy-2-methylpropyl)carbamoyl]-4-hydroxybenzyl}carbamoylmethoxy)-2,3,4,5-tetrahydro-1,2,5-benzothiadiazepin und
1,1-Dioxo-3,3-dibutyl-5-phenyl-7-methylthio-8-(*N*-{(*R*)-1'-phenyl-1'-[*N-*(carboxymethyl)carbamoyl]methyl}carbamoylmethoxy)-2,3,4,5-tetrahydro-1,5-benzoth iazepin
oder ein pharmazeutisch verträgliches Salz davon.

15. Formulierung zur Verwendung nach Anspruch 14, wobei die IBAT-Hemmer-Schicht die Verbindung 1,1-Dioxo-3,3-dibutyl-5-phenyl-7-methylthio-8-(*N-*{(*R*)-α-[*N*-((*S*)-1-carboxypropyl)carbamoyl]-4-hydroxybenzyl}carbamoylmethoxy)-2,3,4,5-tetrahydro-1,2,5-benzothiadiazepin oder ein pharmazeutisch verträgliches Salz davon umfasst.

16. Formulierung zur Verwendung nach einem der Ansprüche 12 bis 15, wobei es sich bei dem Gallensäurebinder um Colestyramin, Colestipol oder Colesevelam handelt.

## Revendications

1. Combinaison comprenant un inhibiteur d'IBAT (transporteur iléal d'acides biliaires) de formule II 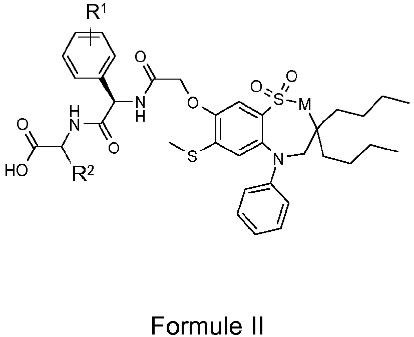 dans laquelle
**M** représente CH₂ ou NH ;
**R¹** représente H ou OH ;
**R²** représente H, CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, -CH₂OCH₃, -CH(OH)CH₃, -CH₂SCH₃, ou -CH₂CH₂SCH₃ ; ou un sel pharmaceutiquement acceptable de celui-ci,
et un séquestrant des acides biliaires,
pour l'administration simultanée, séquentielle ou séparée, dans laquelle le séquestrant des acides biliaires est formulé pour une libération dans le côlon, destinée à être utilisée dans le traitement d'une maladie hépatique choisie dans l'ensemble constitué de la cirrhose biliaire primitive (CBP), de la cholestase intrahépatique familiale progressive (CIFP), du syndrome d'Alagille (SAG), de la cholangite sclérosante primitive (CSP), de la stéatohépatite non alcoolique (SHNA) et du prurit de maladie hépatique cholestatique.

2. Combinaison destinée à être utilisée selon la revendication 1, la maladie hépatique étant la cirrhose biliaire primitive (CBP).

3. Combinaison destinée à être utilisée selon la revendication 1, la maladie hépatique étant la cholestase intrahépatique familiale progressive (CIFP).

4. Combinaison destinée à être utilisée selon la revendication 1, la maladie hépatique étant le syndrome d'Alagille (SAG).

5. Combinaison destinée à être utilisée selon la revendication 1, la maladie hépatique étant la cholangite sclérosante primitive (CSP).

6. Combinaison destinée à être utilisée selon la revendication 1, la maladie hépatique étant la stéatohépatite non alcoolique (SHNA).

7. Combinaison destinée à être utilisée selon la revendication 1, la maladie hépatique étant le prurit de maladie hépatique cholestatique.

8. Combinaison destinée à être utilisée selon l'une quelconque des revendications 1 à 7, laquelle combinaison est conçue pour administrer l'inhibiteur d'IBAT dans l'intestin grêle et le séquestrant des acides biliaires dans le côlon.

9. Combinaison destinée à être utilisée selon l'une quelconque des revendications 1 à 8, dans laquelle le composé de formule II est choisi dans l'ensemble consistant en :
1,1-dioxo-3,3-dibutyl-5-phényl-7-méthylthio-8-(*N*-{(R)-α-[*N-*(carboxyméthyl)carbamoyl]benzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[N'-((S)-1-carboxyéthyl)carbamoyl]benzyl}carbamoylméthoxy)-2,3,4,5-tétrahydro-1,5-benzothiazépine ;
1,1-dioxo-3,3-dibutyl-5-phényl-7-méthylthio-8-(*N*-{(R)-α-[*N*-((S)-1-carboxypropyl)carbamoyl]benzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((R)-1-carboxy-2-méthylthioéthyl)carbamoyl]benzyl}carbamoylméthoxy)-2,3,4,5-tétrahydro-1,2,5-benzothiadiazépine ,
1,1-dioxo-3,3-dibutyl-5-phényl-7-méthylthio-8-(*N*-{(R)-α-[*N*-((S)-1-carboxypropyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N*-{(R)-α-[*N*-((R)-1-carboxy-2-méthylthio-éthyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((S)-1-carboxy-2-méthylpropyl)carbamoyl]benzyl}carbamoylméthoxy)-2,3,4,5-tétrahydro-1,2,5- benzothiadiazépine ;
1,1-dioxo-3,3-dibutyl-5-phényl-7-méthylthio-8-(*N*-{(R)-α-[*N*-((*S*)-1-carboxy-2-(R)-hydroxypropyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N*-{(R)-α-[*N*-((S)-1-carboxybutyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N*-{(R)-α-[*N*-((S)-1-carboxyéthyl)carbamoyl]benzyl}carbamoylméthoxy)-2,3,4,5-tétrahydro-1,2,5-benzothiadiazépine ,
1,1-dioxo-3,3-dibutyl-5-phényl-7-méthylthio-8-(*N*-{(R)-α-[*N'*-((S)-1-carboxypropyl) carbamoyl]-4-hydroxybenzyl}carbamoylméthoxy)-2,3,4,5-tétrahydro-1,5-benzothiazépine ;
1,1-dioxo-3,3-dibutyl-5-phényl-7-méthylthio-8-(*N*-{(R)-α-[*N*-((S)-1-carboxyéthyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((S)-1-carboxy-2-méthylpropyl)carbamoyl]-4-hydroxybenzyl}carbamoylméthoxy)-2,3,4,5-tétrahydro-1,2,5-benzothiadiazépine ; et
1,1-dioxo-3,3-dibutyl-5-phényl-7-méthylthio-8-(N-{(R)-1'-phényl-1'-[N'-(carboxyméthyl)carbamoyl]méthyl}carbamoylméthoxy)-2,3,4,5-tétrahydro-1,5-benzothiazépine ou un de leurs sels pharmaceutiquement acceptables.

10. Combinaison destinée à être utilisée selon la revendication 10, dans laquelle le composé de formule II est la 1,1-dioxo-3,3-dibutyl-5-phényl-7-méthylthio-8-(*N*-{(R)-α-[*N*-((S)-1-carboxypropyl)carbamoyl]-4-hydroxybenzyl}carbamoylméthoxy)-2,3,4,5-tétrahydro-1,2,5-benzothiadiazépine ou l'un de ses sels pharmaceutiquement acceptables.

11. Combinaison destinée à être utilisée selon l'une quelconque des revendications 1 à 10, dans laquelle le séquestrant des acides biliaires est la cholestyramine, le cholestipol ou le colesevelam.

12. Formulation pharmaceutique comprenant un inhibiteur d'IBAT de formule II 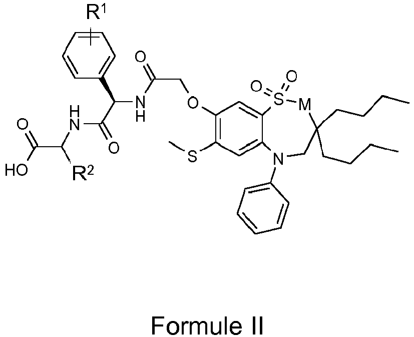 dans laquelle
**M** représente CH₂ ou NH ;
**R¹** représente H ou OH ; et
**R²** représente H, CH₃, -CH₂CH₃, -CH₂CH₂CH₃, -CH₂CH₂CH₂CH₃, -CH(CH₃)₂, -CH₂CH(CH₃)₂, -CH(CH₃)CH₂CH₃, -CH₂OH, -CH₂OCH₃, -CH(OH)CH₃, -CH₂SCH₃, ou -CH₂CH₂SCH₃ ; ou un sel pharmaceutiquement acceptable de celui-ci,
et un séquestrant des acides biliaires,
dans laquelle le séquestrant des acides biliaires se situe dans le coeur et est formulé pour une libération dans le côlon, et dans laquelle l'inhibiteur d'IBAT se situe dans une couche externe formulée pour une libération immédiate ou une libération retardée dans le jéjunum distal ou l'iléon proximal, destinée à être utilisée dans le traitement d'une maladie hépatique choisie dans l'ensemble constitué de la cirrhose biliaire primitive (CBP), de la cholestase intrahépatique familiale progressive (CIFP), du syndrome d'Alagille (SAG), de la cholangite sclérosante primitive (CSP), de la stéatohépatite non alcoolique (SHNA) et du prurit de maladie hépatique cholestatique.

13. Formulation destinée à être utilisée selon la revendication 12, comprenant :
(i) un coeur interne comprenant un séquestrant des acides biliaires ;
(ii) une couche de libération dans le côlon sur le coeur ;
(iii) une couche d'inhibiteur d'IBAT sur ladite couche de libération dans le côlon ; et
(iv) un enrobage externe protecteur.

14. Formulation destinée à être utilisée selon la revendication 12 ou 13, dans laquelle la couche d'inhibiteur d'IBAT comprend un composé choisi dans l'ensemble consistant en :
1,1-dioxo-3,3-dibutyl-5-phényl-7-méthylthio-8-(N-{(R)-α-[N-(carboxyméthyl)carbamoyl]benzyl}carbamoylméthoxy)-2,3,4,5-tétrahydro-1,2,5-benzothiadiazépine ;
1,1-dioxo-3,3-dibutyl-5-phényl-7-méthylthio-8-(N-{(R)-α-[N'-((S)-1-carboxyéthyl)carbamoyl]benzyl}carbamoylméthoxy)-2,3,4,5-tétrahydro-1,5-benzothiazépine ;
1,1-dioxo-3,3-dibutyl-5-phényl-7-méthylthio-8-(*N*-{(R)-α-[*N*-((S)-1-carboxypropyl)carbamoyl]benzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((R)-1-carboxy-2-méthylthioéthyl)carbamoyl]benzyl}carbamoylméthoxy)-2,3,4,5-tétrahydro-1,2,5-benzothiadiazépine ,
1,1-dioxo-3,3-dibutyl-5-phényl-7-méthylthio-8-(*N*-{(R)-α-[*N*-((S)-1-carboxypropyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N*-{(R)-α-[*N*-((R)-1-carboxy-2-méthylthio-éthyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((S)-1-carboxy-2-méthylpropyl)carbamoyl]benzyl}carbamoylméthoxy)-2,3,4,5-tétrahydro-1,2,5-benzothiadiazépine ,
1,1-dioxo-3,3-dibutyl-5-phényl-7-méthylthio-8-(*N*-{(R)-α-[*N*-((S)-1-carboxy-2-(R)-hydroxypropyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N*-{(R)-α-[*N*-((S)-1-carboxybutyl)carbamoyl]- 4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-((*N-*{(R)-α-[*N*-((S)-1-carboxyéthyl)carbamoyl]benzyl}carbamoylméthoxy)-2,3,4,5-tétrahydro-1,2,5-benzothiadiazépine ,
1,1-dioxo-3,3-dibutyl-5-phényl-7-méthylthio-8-(N-{(R)-α-[N'-((S)-1-carboxypropyl)carbamoyl]-4-hydroxybenzyl}carbamoylméthoxy)-2,3,4,5-tétrahydro-1,5-benzothiazépine ;
1,1-dioxo-3,3-dibutyl-5-phényl-7-méthylthio-8-(*N*-{(R)-α-[*N*-((S)-1-carboxyéthyl)carbamoyl]-4-hydroxybenzyl}carbamoylméthoxy)-2,3,4,5-tétrahydro-1,2,5-benzothiadiazépine ;
1,1-dioxo-3,3-dibutyl-5-phényl-7-méthylthio-8-(*N*-{(R)-α-[*N*-((S)-1-carboxy-2-méthylpropyl)carbamoyl]-4-hydroxybenzyl}carbamoylméthoxy)-2,3,4,5-tétrahydro-1,2,5-benzothiadiazépine ; et
1,1-dioxo-3,3-dibutyl-5-phényl-7-méthylthio-8-(N-{(R)-1'-phényl-1'-[N'-(carboxyméthyl)carbamoyl]méthyl}carbamoylméthoxy)-2,3,4,5-tétrahydro-1,5-benzothiazépine ou un de leurs sels pharmaceutiquement acceptables.

15. Formulation destinée à être utilisée selon la revendication 14, dans laquelle la couche d'inhibiteur d'IBAT comprend le composé 1,1-dioxo-3,3-dibutyl-5-phényl-7-méthylthio-8-(*N*-{(R)-α-[*N*-((S)-1-carboxypropyl)carbamoyl]-4-hydroxybenzyl}carbamoylméthoxy)-2,3,4,5-tétrahydro-1,2,5-benzothiadiazépine ou l'un de ses sels pharmaceutiquement acceptables.

16. Formulation destinée à être utilisée selon l'une quelconque des revendications 12 à 15, dans laquelle le séquestrant des acides biliaires est la cholestyramine, le cholestipol ou le colesevelam.
